# FASCICULE DE BREVET EUROPEEN

(11) **EP 3 820 531 B1**
(45) Date de publication et mention de la délivrance du brevet: **04.09.2024**
(21) Numéro de dépôt: 19758433.7
(22) Date de dépôt: 12.07.2019
(51) Int. Cl.: A61K 47/38, A61K 9/51, A61K 35/644

(54) **EXCIPIENT SOUS FORME SOLIDE COMPRENANT UN ENSEMBLE DE FIBRES ALIMENTAIRES VÉGÉTALES NON MODIFIÉES CHIMIQUEMENT**
HILFSSTOFF MIT FESTER FORM, ENTHALTEND NICHT CHEMISCH MODIFIZIERTE PFLANZLICHE BALLASTSTOFFE
EXCIPIENT IN SOLID FORM COMPRISING A SET OF CHEMICALLY UNMODIFIED DIETARY PLANT FIBRES

(30) Priorité: 13.07.2018 FR 1856521
(43) Date de publication de la demande: 19.05.2021
(73) Titulaire: Laboratoires Arkopharma, 06510 Carros (FR)
(72) Inventeur: MENIGAULT-POTIN, Sophie, 06650 LE ROURET (FR); KOGLER, Nadine, 06140 VENCE (FR); SAUVAGE, Julie, 06600 ANTIBES (FR)
(74) Mandataire: Murgitroyd & Company
(86) Numéro de dépôt international: PCT/FR2019/051762
(87) Numéro de publication internationale: WO 2020/012140

(56) Documents cités:
- EP-A1- 2 668 850
- WO-A2-2013/175502
- ES-A1- 2 136 038
- FR-A1- 2 687 309
- FR-A1- 3 037 799
- US-A1- 2013 041 043
- US-A1- 2015 296 851
- US-A1- 2017 027 823

## Description

### Domaine technique

La présente invention concerne le domaine des excipients pharmaceutiques, vétérinaires, pour dispositifs médicaux ou compléments alimentaires et des compositions les comprenant.

### Etat de la technique

Les excipients traditionnellement utilisés dans les compositions à usage pharmaceutique, vétérinaire, pour dispositif médical ou complément alimentaire sont généralement des excipients de synthèse ou sont d'origine naturelle (par exemple d'origine végétale) mais ont été modifiés chimiquement. A ce titre, ils ne sont donc pas utilisables dans des compositions naturelles et/ou conformes aux réglementations idoines en matière de produits biologiques et notamment au règlement CE n° 889/2008 du 5 septembre 2008 (portant sur les modalités d'application du règlement (CE) n° 834/2007 du Conseil relatif à la production biologique et à l'étiquetage des produits biologiques en ce qui concerne la production biologique, l'étiquetage et les contrôles) et ses modifications.

En particulier, depuis des décennies, la demanderesse commercialise avec succès des gélules au sein desquelles est/sont contenue(s) une ou plusieurs substance(s) active(s) d'origine naturelle (notamment d'origine végétale) sous forme pulvérulente ou sous forme d'extrait sec. Ces gélules sont commercialisées sous le nom de marque « Arkogélules^{®} », dont la renommée n'est plus à faire. Ces Arkogélules^{®} sont historiquement les premières gélules dont la tunique a été spécifiquement développée, non pas à partir de gélatine animale, mais sur une base végétale (hydroxypropylméthylcellulose, abrégé en « HPMC » [n° CAS : 9004-65-3]), véritable innovation au moment de la commercialisation de ces gélules. La taille de ces gélules peut varier principalement en fonction du volume total représenté par la préparation à base de substance(s) active(s) d'origine naturelle (en particulier d'origine végétale) sous forme solide et le ou les excipient(s), volume correspondant à la dose requise par la posologie.

Les enveloppes de ces Arkogélules^{®} sont adaptées et destinées à se dissoudre rapidement dans l'estomac du patient/sujet afin d'y libérer la ou les substance(s) active(s) contenue(s) et, ce faisant, permettre leur biodisponibilité.

A minima, la formulation d'une gélule - et notamment d'une Arkogélule^{®} - doit satisfaire aux exigences de fabrication, d'uniformité de la dose posologique, de stabilité, de désintégration et de biodisponibilité. Or, la stabilité de la forme galénique « gélule contenant une ou plusieurs substance(s) active(s) d'origine naturelle » est une problématique majeure d'intérêt au vu de l'hygroscopicité des substances actives entrant dans la gamme Arkogélules^{®}. Cette problématique était jusqu'alors résolue par l'ajout, dans les compositions d'intérêt, d'excipients traditionnels issus ou dérivés de l'industrie pharmaceutique. Plus précisément, il est possible de citer les excipients traditionnels suivants :
- la cellulose microcristalline (n° CAS : 9004-34-6), généralement utilisée en tant qu'agent de charge (et représentant jusqu'à 80% en masse de la composition totale), est considérée comme étant d'origine végétale mais s'avère néanmoins chimiquement modifiée (séparation de la lignine et de l'hémicellulose, puis purification par hydrolyse acide de manière à éliminer la partie de la cellulose amorphe); l'emploi de ladite cellulose microcristalline n'étant de plus pas autorisée par les réglementations idoines en matière de produits biologiques (et notamment règlement CE n° 889/2008 du 5 septembre 2008 et ses modifications) ;
- le stéarate de magnésium (n° CAS : 557-04-0), utilisé en qualité d'agent anti-agglomérant (jusqu'à 2% en masse de la formule totale), n'est également pas autorisé par les réglementations idoines en matière de produits biologiques (et notamment le règlement CE n° 889/2008 du 5 septembre 2008 et ses modifications) ; et
- la ou les silices, dont l'utilisation comme agents d'écoulement et/ou agents anti-agglomérants (jusqu'à 3% de la formule totale) est à proscrire au sein de produits biologiques, notamment en raison de leur voie d'obtention synthétique.

Par conséquent, même si les excipients traditionnels susvisés possèdent des propriétés physico-chimiques et pharmaco-techniques reconnues, il existe un besoin de mettre au point des excipients « plus naturels », et en particulier compatibles avec les réglementations idoines en matière de produits biologiques (et notamment le règlement CE n° 889/2008 du 5 septembre 2008 et ses modifications), à savoir des excipients naturels (par exemple de nature végétale) ou d'origine naturelle (par exemple d'origine végétale) non modifiés chimiquement (à savoir n'ayant subi aucune modification/transformation chimique ou biochimique ; de préférence uniquement obtenus par un ou plusieurs procédés mécaniques, permettant par exemple d'obtenir des produits pulvérulents: parés, tranchés, hachés, épluchés, moulus, broyés, décortiqués, séchés, etc.) possédant des propriétés physico-chimiques et pharmaco-techniques voisines de - ou sensiblement équivalentes à - celles des excipients mentionnés supra, issus ou dérivés de l'industrie pharmaceutique.

En outre, le mode de fabrication préférentiel utilisé pour les susdites Arkogélules^{®} est une homogénéisation directe des poudres, ce qui implique que les propriétés pharmaco-techniques du mélange avant mise en forme ne sont pas conférées par une étape de procédé industriel proprement dite, par exemple de type granulation humide ou sèche, mais bien par l'ensemble des propriétés des ingrédients entrant dans la composition. Ce choix industriel se justifie notamment dans un souci de préservation des substances actives d'une exposition à l'humidité et à la chaleur, et est privilégié d'un point de vue industriel dans un souci économique car ces étapes sont très coûteuses en termes de temps et d'énergies consommées. Par conséquent, les susdits excipients « plus naturels » (de préférence compatibles avec les réglementations idoines en matière de produits biologiques) doivent également permettre, en lien avec la/les substance(s) active(s) d'origine naturelle avec laquelle/lesquelles ils sont mélangés, d'obtenir directement les propriétés pharmaco-techniques souhaitées (par exemple dans le cadre d'une homogénéisation directe des poudres), notamment en termes d'écoulement et de stabilité physico-chimique de la composition pendant toute la durée de fabrication et jusqu'à péremption du produit, sans nécessiter une étape de procédé industriel à proprement parler, par exemple de type granulation humide ou sèche.

La demande de brevet publiée sous la référence US 2017/027823 A1 enseigne notamment des capsules, utilisables dans des compositions cosmétiques et/ou pharmaceutiques, comprenant un agent de charge, ce dernier étant, selon un mode de réalisation, une fibre végétale choisie dans le groupe constitué par les fibres de pomme, les fibres de bambou, les fibres d'avoine, les fibres de pois, les fibres de pomme de terre, les fibres de blé et leurs mélanges.

Les demandes de brevet respectivement publiées sous les références US 2013/041043 A1 et WO 2013/175502 A2 apparaissent avoir en commun d'envisager l'utilisation d'un excipient issu de fenugrec (*Trigonella foenum-graceum*) (et plus particulièrement de graines de fenugrec) ; étant précisé que ledit excipient est utilisé, selon WO 2013/175502 A2, au sein d'une formulation adaptée pour une administration par voie mucosale.

Dans le cadre de ses recherches, la demanderesse a découvert que ces objectifs pouvaient être atteints en utilisant un ensemble de fibres alimentaires végétales non modifiées chimiquement, ledit ensemble de fibres alimentaires comprenant :
- un pourcentage massique de fibres solubles dans l'eau inférieur à 20%, de préférence inférieur ou égal à 15%, par exemple inférieur ou égal à 10%, par rapport à la masse totale de l'excipient, et
- un pourcentage massique de fibres insolubles dans l'eau supérieur à 40%, de préférence supérieur ou égal à 45%, par rapport à la masse totale de l'excipient ;
ledit excipent présentant avantageusement un pourcentage massique en eau inférieur ou égal à 8% par rapport à la masse totale de l'excipient.

### Exposé de l'invention

Par conséquent, l'invention a pour objet une composition à usage pharmaceutique, vétérinaire, pour dispositif médical ou pour complément alimentaire, ladite composition se présentant sous forme solide, de préférence sous forme pulvérulente, et comprenant :
- au moins une substance active, de préférence d'origine naturelle, préférablement d'origine naturelle végétale ou d'origine naturelle issue de la ruche, et
- au moins un excipient à usage pharmaceutique, vétérinaire, pour dispositif médical (à usage humain ou animal) ou pour complément alimentaire (à usage humain ou animal), ledit excipient se présentant sous forme solide et comprenant un ensemble de fibres alimentaires végétales non modifiées chimiquement, dans un pourcentage massique supérieur ou égal à 50% (préférablement supérieur ou égal à 55%, avantageusement supérieur ou égal à 60%) par rapport à la masse totale de l'excipient, ledit ensemble de fibres alimentaires comprenant, consistant essentiellement en, ou consistant en :
   - un pourcentage massique de fibres solubles dans l'eau inférieur à 20%, de préférence inférieur ou égal à 15%, par exemple inférieur ou égal à 10%, par rapport à la masse totale de l'excipient, et
   - un pourcentage massique de fibres insolubles dans l'eau supérieur à 40%, de préférence supérieur ou égal à 45%, par rapport à la masse totale de l'excipient ;

   ledit excipent présentant avantageusement un pourcentage massique en eau inférieur ou égal à 8% par rapport à la masse totale de l'excipient ;
   ledit excipient se présentant sous forme pulvérulente, ledit excipient sous forme pulvérulente étant sous forme de particules de taille micronique telles que des particules micronisées, lesdites particules de taille micronique ayant une granulométrie inférieure ou égale à 1000 µm, préférablement inférieure à 1000 µm, et se situant préférentiellement entre environ 1 µm et environ 250 µm.

L'excipient aux fins de l'invention permet de résoudre tout ou partie des problèmes susvisés et/ou atteindre tout ou partie des objectifs susmentionnés. En particulier, ledit excipient est un excipient « plus naturel » que les excipients traditionnellement utilisés (issus et/ou dérivés de l'industrie pharmaceutique), potentiellement compatible avec les réglementations idoines en matière de produits biologiques (et notamment le règlement CE n° 889/2008 du 5 septembre 2008 et ses modifications) et présentant des propriétés pharmaco-techniques voisines de - ou sensiblement équivalentes à - celles des excipients traditionnellement utilisés.

De surcroît, cet excipient est tout à fait compatible avec la technique d'homogénéisation directe des poudres, technique privilégiée par la demanderesse pour la fabrication de ses Arkogélules^{®} pour les raisons exposées supra.

Selon un mode de réalisation préféré, l'ensemble de fibres alimentaires susvisé comprend une teneur en eau et/ou une valeur d'Aw de départ basse, avantageusement les deux (teneur en eau inférieure à 8% et/ou valeur d'Aw inférieure à 0,5, avantageusement les deux).

Selon un mode de réalisation préféré, ledit ensemble de fibres alimentaires comprend, consiste essentiellement en, ou consiste en, des fibres de plante(s) (de préférence de légumineuse(s)), de fruit(s) et/ou des fibres de céréale(s), de préférence des fibres de pomme (*Malus domestica* Borkh), d'avoine (*Avena sativa L.*), d'aronia (*Aronia melanocarpa* (Michaux.) S. Elliott), de cassis (*Ribes nigrum L*.), de pois (*Pisum sativum L.*) et/ou de sarrasin (*Fagopyrum esculentum* Moench). Selon un mode de réalisation particulièrement préféré, ledit ensemble de fibres alimentaires comprend, consiste essentiellement en, ou consiste en, des fibres de pomme et/ou de sarrasin.

Selon un mode de réalisation, les fibres alimentaires végétales non modifiées chimiquement du susdit ensemble de fibres alimentaires proviennent d'une même source de fibres végétales. De préférence, elles consistent en des fibres alimentaires d'une même espèce végétale (origine commune). Par exemple, ces fibres alimentaires végétales non modifiées chimiquement proviennent de la même espèce de plante (de préférence de légumineuse), de fruit ou de céréale. Selon un aspect préféré de ce mode de réalisation, ces fibres alimentaires végétales non modifiées chimiquement sont des fibres de pomme, d'avoine, d'aronia, de cassis, de pois ou de sarrasin.

Selon un autre mode de réalisation, les fibres alimentaires végétales non modifiées chimiquement du susdit ensemble de fibres alimentaires consistent en un mélange de fibres alimentaires d'une pluralité de végétaux (à savoir provenant d'au moins deux sources différentes de fibres végétales). Par exemple, ce mélange de fibres alimentaires végétales non modifiées chimiquement provient :
i) d'au moins deux plantes différentes (par exemple de deux types de légumineuse), d'au moins deux fruits différents, d'au moins deux céréales différentes, ou
ii) d'une combinaison d'au moins deux sources différentes de fibres végétales sélectionnées parmi une plante (de préférence une légumineuse), un fruit et une céréale.

Selon un aspect de ce mode de réalisation, ledit mélange de fibres alimentaires - végétales non modifiées chimiquement - de ladite pluralité de végétaux comprend :
- un pourcentage massique de fibres solubles dans l'eau inférieur à 20%, de préférence inférieur ou égal à 15%, par exemple inférieur ou égal à 10%, par rapport à la masse totale de l'excipient, et
- un pourcentage massique de fibres insolubles dans l'eau supérieur à 40%, de préférence supérieur ou égal à 45%, par rapport à la masse totale de l'excipient.

Selon un autre aspect de ce mode de réalisation, l'ensemble de fibres alimentaires - végétales non modifiées chimiquement - de chaque végétal de ladite pluralité de végétaux (à savoir provenant de chacun desdits végétaux) comprend :
- un pourcentage massique de fibres solubles dans l'eau inférieur à 20%, de préférence inférieur ou égal à 15%, par exemple inférieur ou égal à 10%, par rapport à la masse totale de l'excipient, et
- un pourcentage massique de fibres insolubles dans l'eau supérieur à 40%, de préférence supérieur ou égal à 45%, par rapport à la masse totale de l'excipient.

De préférence, les susdites fibres alimentaires végétales non modifiées chimiquement sont obtenues par un procédé comprenant, consistant essentiellement en, ou consistant en :
a) une étape de préparation de la matière première (par exemple, plante(s), fruit(s) et/ou céréale(s)), par exemple par pressage, séchage ou stabilisation thermique,
b) une ou plusieurs étape(s) de broyage de la matière première préparée conformément à l'étape a),
c) une ou plusieurs étape(s) de tamisage de la matière première broyée obtenue à l'étape b), de manière à obtenir des fibres alimentaires végétales non modifiées chimiquement ayant une granulométrie inférieure à 1000 µm et se situant préférentiellement entre 1 et 250 µm (cette granulométrie étant particulièrement préférée dans la mesure où elle permet d'obtenir des poudres fines ; la faible granulométrie de ces poudres permettant d'obtenir une surface d'échange importante et, par conséquent, une capacité d'absorption d'autant plus importante et de s'adsorber relativement aisément à la surface des particules de substance(s) active(s) à protéger grâce aux forces régissant le comportement des particules de faible taille constituant ces poudres fines (poudres de faible granulométrie).

Ledit excipient se présente sous forme pulvérulente, ledit excipient sous forme pulvérulente étant sous forme de particules de taille micronique telles que des particules micronisées, lesdites particules de taille micronique ayant une granulométrie inférieure ou égale à 1000 µm, préférablement inférieure à 1000 µm, et se situant préférentiellement entre environ 1 µm et environ 250 µm (de préférence entre 1 µm et 250 µm) ; cette dernière granulométrie étant en effet particulièrement préférée dans la mesure où elle permet d'obtenir des poudres fines. En effet, et tel qu'indiqué précédemment, la faible granulométrie de ces « poudres fines » permet :
i) d'obtenir une surface d'échange importante et, par conséquent, une capacité d'absorption d'autant plus importante, et
ii) de s'adsorber relativement aisément à la surface des particules de substance(s) active(s) à protéger grâce aux forces régissant le comportement des particules de faible taille constituant ces poudres fines (poudres de faible granulométrie).

Selon un mode de réalisation particulièrement préféré, ledit excipient sous forme pulvérulente présente la distribution granulométrique suivante :
- Dv10 < 20 µm (de préférence < 15 µm),
- Dv50 < à 100 µm (de préférence < à 50 µm), et/ou
- Dv90 < à 250 µm (de préférence < à 200 µm) ;
de manière préférée ladite distribution granulométrique étant la suivante :
- Dv90 < à 250 µm (de préférence < à 200 µm), et
- Dv50 < à 100 µm (de préférence < à 50 µm) et/ou Dv10 < 20 µm (de préférence < 15 µm) ;
de manière particulièrement préférée ladite distribution granulométrique étant la suivante :
- Dv90 < à 250 µm (de préférence < à 200 µm), et
- Dv50 < à 100 µm (de préférence < à 50 µm) et Dv10 < 20 µm (de préférence < 15 µm).

Tel que souligné précédemment, la taille relativement faible des particules de l'excipient aux fins de l'invention (celui-ci se trouve sous forme pulvérulente) permet auxdites particules d'excipient de présenter une surface spécifique importante, donc une surface d'échange importante, et, de surcroît, de s'adsorber relativement aisément à la surface des particules de substance(s) active(s) à protéger grâce aux forces régissant le comportement des particules de faible taille (particules fines).

L'invention a également pour objet l'utilisation de l'excipient aux fins de l'invention en tant qu'agent de charge, agent stabilisant et/ou agent anti-agglomérant, de préférence en tant qu'agent anti-agglomérant, avantageusement en tant qu'agent anti-agglomérant sélectionné parmi les agents protecteurs contre l'humidité, dans une composition pharmaceutique, vétérinaire, un dispositif médical ou un complément alimentaire (ou dans une composition à usage pharmaceutique, vétérinaire, pour dispositif médical ou pour complément alimentaire).

L'invention concerne une composition à usage pharmaceutique, vétérinaire, pour dispositif médical (à usage humain ou animal) ou pour complément alimentaire (à usage humain ou animal), ladite composition se présentant sous forme solide, de préférence sous forme pulvérulente, et comprenant, consistant essentiellement en, ou consistant en :
- au moins une substance active, de préférence d'origine naturelle, préférablement d'origine naturelle végétale ou issue de la ruche (en quantité efficace), et
- au moins un excipient à usage pharmaceutique, vétérinaire, pour dispositif médical ou pour complément alimentaire ;

ledit excipient se présentant sous forme solide et comprenant un ensemble de fibres alimentaires végétales non modifiées chimiquement, dans un pourcentage massique supérieur ou égal à 50% par rapport à la masse totale de l'excipient, ledit ensemble de fibres alimentaires comprenant :
   - un pourcentage massique de fibres solubles dans l'eau inférieur à 20%, de préférence inférieur ou égal à 15%, par exemple inférieur ou égal à 10%, par rapport à la masse totale de l'excipient, et
   - un pourcentage massique de fibres insolubles dans l'eau supérieur à 40%, de préférence supérieur ou égal à 45%, par rapport à la masse totale de l'excipient ;
ledit excipent présentant avantageusement un pourcentage massique en eau inférieur ou égal à 8% par rapport à la masse totale de l'excipient ;
ledit excipient se présentant sous forme pulvérulente, ledit excipient sous forme pulvérulente étant sous forme de particules de taille micronique telles que des particules micronisées, lesdites particules de taille micronique ayant une granulométrie inférieure ou égale à 1000 µm, préférablement inférieure à 1000 µm, et se situant préférentiellement entre environ 1 µm et environ 250 µm.

Selon un mode de réalisation, ledit excipient comprend :
i) une teneur en fibres alimentaires - végétales non modifiées chimiquement - solubles dans l'eau inférieure à 10% de la masse totale de ladite composition, de préférence inférieure ou égale à 6% de la masse totale de ladite composition, préférablement inférieure ou égale à 5% de la masse totale de ladite composition, avantageusement inférieure ou égale à 4% de la masse totale de ladite composition (et préférablement comprise entre 0,1 et 3% de la masse totale de ladite composition), et/ou
ii) une teneur en fibres alimentaires - végétales non modifiées chimiquement - insolubles dans l'eau supérieure à 2% de la masse totale de ladite composition, de préférence supérieure ou égale à 5% de la masse totale de ladite composition, préférablement supérieure ou égale à 8% de la masse totale de ladite composition ;
avantageusement les deux.

Selon un mode de réalisation, la composition selon l'invention comprend une pluralité (à savoir au moins deux) d'excipients aux fins de l'invention.

Selon un mode de réalisation, la composition selon l'invention comprend ledit au moins un excipient dans un pourcentage massique inférieur à 50%, de préférence inférieur à 45%, et avantageusement inférieur à 40%, par rapport à la masse totale de la composition.

De préférence, ladite au moins une substance active (de préférence d'origine naturelle, préférablement d'origine naturelle végétale ou issue de la ruche) est une substance hygroscopique.

Lorsque la composition selon l'invention se présente sous forme pulvérulente (mode de réalisation préféré de l'invention), les particules d'excipient ont une granulométrie inférieure ou équivalente à la granulométrie des particules de la susdite au moins une substance active. Ceci permet aux particules d'excipient de granulométrie inférieure ou équivalente (présentant donc une surface spécifique importante) de s'adsorber relativement aisément à la surface des particules de substance(s) active(s) à protéger.

L'invention concerne également une composition pharmaceutique, une composition vétérinaire, un dispositif médical ou un complément alimentaire comprenant l'excipient aux fins de l'invention et/ou la composition selon l'invention.

Selon un mode de réalisation préféré, ladite au moins une substance active est une préparation à base de plante(s) sous forme de poudre et/ou une substance active d'origine naturelle issue de la ruche telle que le miel, la gelée royale ou la propolis sous forme de poudre ; de manière préférée ladite poudre ayant une granulométrie inférieure à 1000 µm et se situant préférentiellement entre 1 et 250 µm.

Un objet de l'invention concerne également une forme galénique solide comprenant la composition selon l'invention, ladite forme galénique solide étant sélectionnée parmi :
- un comprimé, tel qu'un comprimé à avaler, à croquer, effervescent, orodispersible,
- une pastille,
- une poudre, telle qu'une poudre en sachet, en stick, vrac, une gélule ;
ladite forme galénique solide étant préférablement sélectionnée parmi un comprimé ou une gélule ; de manière particulièrement préférée ladite forme galénique solide étant une gélule.

L'invention concerne également un médicament à usage humain ou animal (de préférence à base de substance(s) active(s) d'origine naturelle végétale(s) ou de substance(s) active(s) d'origine naturelle issue(s) de la ruche), un dispositif médical (à usage humain ou animal ; de préférence à base de substance(s) active(s) d'origine naturelle végétale(s) ou de substance(s) active(s) d'origine naturelle issue(s) de la ruche), ou un complément alimentaire (à usage humain ou animal ; de préférence à base de substance(s) active(s) d'origine naturelle végétale(s) ou de substance(s) active(s) d'origine naturelle issue(s) de la ruche), comprenant la composition selon l'invention ou la forme galénique solide selon l'invention.

L'invention a également pour objet un médicament à usage humain ou animal à base de plante(s) ou de substance(s) active(s) d'origine naturelle issue(s) de la ruche, un dispositif médical à base de plante(s) ou de substance(s) active(s) d'origine naturelle issue(s) de la ruche, ou un complément alimentaire à base de plante(s) ou de substance(s) active(s) d'origine naturelle issue(s) de la ruche, comprenant la composition selon l'invention ou la forme galénique solide selon l'invention.

Un autre objet de l'invention concerne l'utilisation d'un ensemble de fibres alimentaires végétales non modifiées chimiquement pour la préparation d'un excipient à usage pharmaceutique, vétérinaire, pour dispositif médical ou pour complément alimentaire se présentant sous forme solide, ledit excipent présentant avantageusement un pourcentage massique en eau inférieur ou égal à 8% par rapport à la masse totale de l'excipient, ledit ensemble de fibres alimentaires comprenant, consistant essentiellement en, ou consistant en :
- un pourcentage massique de fibres solubles dans l'eau inférieur à 20%, de préférence inférieur ou égal à 15%, par exemple inférieur à 10%, par rapport à la masse totale de l'excipient, et
- un pourcentage massique de fibres insolubles dans l'eau supérieur à 40%, de préférence supérieur ou égal à 45%, par rapport à la masse totale de l'excipient ;

ledit ensemble de fibres alimentaires étant utilisé dans un pourcentage massique supérieur ou égal à 50% (de préférence à 55%, avantageusement à 60%) par rapport à la masse totale de l'excipient ;
ledit excipient se présentant sous forme pulvérulente, ledit excipient sous forme pulvérulente étant sous forme de particules de taille micronique telles que des particules micronisées, lesdites particules de taille micronique ayant une granulométrie inférieure ou égale à 1000 µm, préférablement inférieure à 1000 µm, et se situant préférentiellement entre environ 1 µm et environ 250 µm.

L'invention a également pour objet l'utilisation d'un excipient aux fins de l'invention pour la préparation :
- d'une composition à usage pharmaceutique, vétérinaire, pour dispositif médical (à usage humain ou animal) ou pour complément alimentaire (à usage humain ou animal) se présentant sous forme solide, de préférence sous forme pulvérulente, et/ou
- d'une composition pharmaceutique, d'une composition vétérinaire, d'un dispositif médical (à usage humain ou animal) ou d'un complément alimentaire (à usage humain ou animal) selon l'invention se présentant sous forme solide, de préférence sous forme pulvérulente.

Un avantage additionnel de l'invention réside dans le fait que les excipients aux fins de l'invention peuvent être utilisés au sein des compositions pharmaceutiques, vétérinaires, dispositifs médicaux ou compléments alimentaires d'intérêt dans des concentrations similaires ou différentes à celles des excipients traditionnellement employés (excipients de synthèse ou d'origine naturelle modifiés chimiquement). En tout état de cause, le choix et la quantité d'excipient(s) selon la présente description demeurent dépendants des propriétés pharmaco-techniques des substances actives en lien avec la forme galénique à développer, notamment en termes d'écoulement et de stabilité physico-chimique de la composition pendant toute la durée de fabrication et jusqu'à péremption du produit.

En outre, les excipients selon la présente description peuvent être inclus au sein des compositions d'intérêt sans nécessiter de modification des procédés industriels de fabrication existants, ce qui représente un avantage significatif.

Les excipients selon la présente description permettent donc de reformuler, de manière relativement aisée, les compositions existantes, et en particulier les compositions comprenant des substances actives hygroscopiques telles que certaines poudres de plante(s) ou certains extraits végétaux secs ou encore certains produits issus de la ruche. Qui plus est, et tel que souligné précédemment, une telle reformulation peut être effectuée sans modifications des procédés industriels de fabrication existants, modifications bien évidemment coûteuses en temps homme mais également sur le plan pécuniaire.

### Définitions

***Excipient à usage pharmaceutique, vétérinaire, pour dispositif médical ou pour complément alimentaire.*** Substance ou composition naturelle ou de synthèse sans activité thérapeutique entrant dans une composition pharmaceutique, vétérinaire, un dispositif médical ou un complément alimentaire, et jouant un rôle important afin d'en améliorer les propriétés pharmaco-techniques. Les excipients agissent par exemple pour protéger, maintenir et/ou améliorer la stabilité de la formulation, pour permettre la mise en forme unitaire, pour améliorer des caractéristiques organoleptiques (goût, aspect, etc.), afin d'en faciliter l'observance par le patient ou le sujet, ou améliorer la biodisponibilité des substances actives.

***Dispositif(s) médical(aux) :*** Tout instrument, appareil, équipement, matière ou autre article, utilisé seul ou en association, y compris le logiciel nécessaire pour le bon fonctionnement de celui-ci, destiné par le fabricant à être utilisé chez l'homme à des fins :
- de diagnostic, de prévention, de contrôle, de traitement ou d'atténuation d'une maladie,
- de diagnostic, de contrôle, de traitement, d'atténuation ou de composition d'une blessure ou d'un handicap,
- d'étude ou de remplacement ou modification de l'anatomie ou d'un processus physiologique,
- de maîtrise de la conception,
et dont l'action principale voulue dans ou sur le corps humain n'est pas obtenue par des moyens pharmacologiques ou immunologiques ni par métabolisme, mais dont la fonction peut être assistée par de tels moyens.

***Propriétés pharmaco-techniques*** : propriétés techniques inhérentes aux excipients utilisés pour la formulation de produits pharmaceutiques, vétérinaires, de dispositifs médicaux ou encore de compléments alimentaires, garantissant l'aptitude à assurer la fonction pour laquelle il leur est fait appel. Ces propriétés servent notamment à corriger les défauts de fonctionnalité des substances actives, permettant ainsi de répondre aux exigences des procédés de fabrication des différentes formes galéniques concernées.

Les propriétés pharmaco-techniques sont multiples et leur intérêt dépend de la forme galénique. Elles sont mises en évidence par des méthodes de caractérisation appropriées et influent sur les décisions prises en termes de formulation et de procédés industriels. Par exemple, on note parmi ces propriétés l'écoulement, l'aptitude au tassement, et des propriétés de compressibilité.

***Fibres alimentaires**.* Au sens de la présente invention, la définition de « fibres alimentaires » est conforme la définition présentée dans le règlement (UE) n° 1169-2011 du Parlement européen et du Conseil du 25 octobre 2011. Cette définition est la suivante : « Fibres alimentaires » : les polymères glucidiques composés de trois unités monomériques ou plus, qui ne sont ni digérés ni absorbés dans l'intestin grêle humain et appartiennent à l'une des catégories suivantes :
- polymères glucidiques comestibles, présents naturellement dans la denrée alimentaire telle qu'elle est consommée,
- polymères glucidiques comestibles qui ont été obtenus à partir de matières premières alimentaires brutes par des moyens physiques, enzymatiques ou chimiques et ont un effet physiologique bénéfique démontré par des données scientifiques généralement admises,
- polymères glucidiques comestibles synthétiques qui ont un effet physiologique bénéfique démontré par des données scientifiques généralement admises.

***Fibres alimentaires végétales.*** Par « fibres alimentaires végétales », l'on entend, au sens de la présente invention, des fibres alimentaires comestibles obtenues à partir de végétaux et, en particulier, de graines, de tiges, de feuilles, d'écorces, de racines ou de fruits.

***Fibres alimentaires végétales non modifiées chimiquement.*** Selon la présente invention, et afin de permettre notamment la mise au point d'excipients « plus naturels » et, de préférence compatibles avec les réglementations idoines en matière de produits biologiques, les fibres alimentaires végétales susvisées n'ont subi aucune modification/transformation chimique. Au sens de la présente invention, de telles modifications/transformations chimiques englobent, au sens large, des modifications/transformations biochimiques telles que des modifications/transformations enzymatiques.

Selon un mode de réalisation particulièrement préféré, les susdites fibres alimentaires végétales non modifiées chimiquement sont obtenues uniquement par un ou plusieurs procédé(s) mécanique(s) (permettant par exemple d'obtenir des produits parés, tranchés, hachés, épluchés, moulus, broyés, décortiqués, séchés...).

***Détermination du caractère soluble*/*****insoluble dans l'eau des fibres alimentaires végétales non modifiées chimiquement.*** La solubilité des fibres dans l'eau gouverne leur classification en solubles/insolubles. La solubilité repose sur la présence de nombreuses fonctions alcool capables d'interagir avec l'eau, mais n'est pas pour autant en lien avec leur absorption au niveau digestif. Le principe de la méthode d'analyse du caractère soluble/insoluble des fibres (méthode AOAC : « enzymatic-weight method according to AOAC 991.43, AACC 32-07 ») est le suivant :
« Après délipidation, un échantillon double du produit est traité par autoclavage en vue de la gélatinisation de l'amidon puis soumis successivement à l'action de l'amyloglucosidase et de la trypsine. Le résidu de ces opérations représente les fibres alimentaires insolubles. Le surnageant obtenu lors de deux traitements enzymatiques est traité à l'éthanol afin de précipiter les fibres solubles. Les fibres insolubles et les fibres solubles sont filtrées, lavées, séchées puis pesées. Sur l'un des essais, une calcination est réalisée afin d'apprécier la teneur en substances minérales ; sur l'autre le taux de protéines est déterminé. La quantité de fibres totales est donnée par la somme des quantités de fibres solubles et des fibres insolubles, chacune de ces valeurs étant diminuée de la somme des teneurs en matières minérales et en protéines. »

***Granulométrie**.* La granulométrie est définie comme la répartition statistique des particules qui composent la poudre en fonction de leurs dimensions (taille et forme des particules élémentaires). La taille des particules est toujours exprimée par un diamètre. Lorsque la particule a la forme d'une sphère, le diamètre de celle-ci est facilement défini ; en revanche, lorsque la forme de la particule est très différente de celle d'une sphère (forme irrégulière, poreuse...), la notion de diamètre équivalent à une sphère est utilisée.

***Diamètre en volume (Dv).*** Le Dv est le diamètre de la sphère qui aurait le même volume que la particule considérée. Il existe de nombreux diamètres équivalents différents utilisés en granulométrie, mais le diamètre en volume (Dv) est le plus courant.

***Dv50.*** La distribution granulométrique ou distribution en taille des particules Dv50 est un des diamètres caractéristiques également connu comme le diamètre médian ou valeur médiane de la distribution granulométrique, et correspond au diamètre des particules (µm) en-dessous duquel se situe 50% du volume des particules sur la courbe de distribution exprimée en fréquence cumulée. Par exemple, si Dv50 = 10µm, 50% du volume des particules de l'échantillon ont un diamètre inférieur à 10µm et 50% du volume des particules ont un diamètre supérieur à 10µm. Les deux autres diamètres caractéristiques usuellement utilisés pour décrire la distribution granulométrique des poudres sont Dv10 et Dv90.

***Dv10.*** Correspond au diamètre (µm) en-dessous duquel se situe 10% du volume des particules.

***Dv90.*** Correspond au diamètre (µm) en-dessous duquel se situe 90% du volume des particules.

***Particules de taille micronique.*** Particules obtenues par une opération mécanique de broyage permettant la réduction d'un solide en petites particules, en l'occurrence l'obtention d'une poudre très fine de l'ordre du micron (granulométrie d'environ 1 à 1000 µm). De préférence, lesdites particules sont des particules micronisées, à savoir obtenues par une opération de pulvérisation appelée « micronisation », permettant la réduction d'un solide en petites particules en l'occurrence l'obtention d'une poudre très fine de l'ordre du micron (granulométrie d'environ 1 à 1000 µm).

***Agents de charge.*** Les « agents de charge » sont des excipients qui jouent un rôle de remplissage lorsque la quantité de substance(s) active(s) est insuffisante par rapport au volume de la forme galénique à développer ou lorsque les propriétés pharmaco-techniques de ladite/desdites substance(s) active(s) ne sont pas adaptées. Les agents de charge sont utilisés seuls ou en mélange, et choisis en fonction de leurs propriétés pharmaco-techniques secondaires (ex : pouvoir adsorbant, influence sur la dureté, influence sur l'écoulement, solubilité...). A titre d'exemple, l'un des agents de charge le plus fréquemment utilisé est la cellulose microcristalline.

***Agents stabilisants.*** Les « agents stabilisants » (ou plus simplement «stabilisants») sont des substances qui, ajoutées à une denrée alimentaire, permettent de maintenir son état physico-chimique. Les agents stabilisants comprennent les substances qui permettent de maintenir la dispersion homogène de deux ou plusieurs substances non miscibles dans une denrée alimentaire, les substances qui stabilisent, conservent ou intensifient.

***Agents anti-agglomérants.*** Les « agents anti-agglomérants » (ou plus simplement « anti-agglomérants ») sont des excipients dont au moins une des propriétés pharmaco-techniques est de limiter l'agglutination des particules entre elles.

***Agents protecteurs contre l'humidité.*** Les « agents protecteurs contre l'humidité » sont des agents anti-agglomérants permettant de protéger les substances actives hygroscopiques par absorption ou absorption préférentielle de l'eau, sans changer d'état, et permettant de maintenir la pulvérulence de la composition ainsi que les caractéristiques physico-chimiques initiales des substances actives.

***Substance hygroscopique.*** Substance qui a tendance à absorber l'humidité de l'air, par absorption ou par adsorption. Les excipients aux fins de l'invention sont particulièrement utiles notamment en tant qu'agents anti-agglomérants (et en particulier en tant qu'agents protecteurs contre l'humidité) lorsqu'ils sont incorporés dans des compositions solides comprenant des poudres de plante(s) hygroscopique(s).

***Complément alimentaire.*** Composition à usage humain ou animal (de préférence à usage humain) dont le but est de compléter le régime normal et qui constitue une source concentrée de nutriments ou d'autres substances ayant un effet nutritionnel ou physiologique, seuls ou combinés, commercialisés sous forme de doses, à savoir les formes de présentation telles que les gélules, les pastilles, les comprimés, les pilules et autres formes similaires, ainsi que les sachets de poudre, les ampoules de liquide, les flacons munis d'un compte-gouttes et les autres formes analogues de préparations liquides ou en poudre destinées à être prises en unités mesurées de faible quantité. La composition d'un « complément alimentaire » selon l'invention est à visée nutritionnelle.

***Substance active.*** Une substance active est une substance qui possède des propriétés bénéfiques pour l'organisme ayant par exemple un effet nutritionnel ou physiologique, ou une activité thérapeutique.

***Substance active d'origine naturelle.*** Une substance active d'origine naturelle est une substance active présente telle quelle dans la nature et non traitée, ou alors traitée sans être modifiée chimiquement. La structure moléculaire de la substance active doit notamment demeurer inchangée. Ces substances actives d'origine naturelle peuvent être par exemple d'origine végétale, minérale, un produit de fermentation ou encore des substances actives d'origine naturelle issues de la ruche.

***Substances actives d'origine naturelle issues de la ruche.*** Les substances actives d'origine naturelle issues de la ruche sont le miel, la gelée royale, la propolis, utilisées en l'état ou des préparations à base de miel, la gelée royale, la propolis.

***Substances actives d'origine naturelle végétale.*** Les substances actives d'origine naturelle végétale sont essentiellement :
- des plantes, parties de plantes, algues, champignons, lichens ; entiers, fragmentés ou brisés, utilisés en l'état, soit le plus souvent sous forme desséchée, soit à l'état frais ; et/ou
- des préparations à base de plantes, algues, champignons, lichens, par exemple, des extraits, des huiles essentielles, des jus d'expression, des exsudats ayant subi un traitement, ou des substances végétales ayant subi une opération de réduction de taille pour des applications spécifiques (par exemple, divisées pour des tisanes ou pulvérisées pour mise en forme gélule).

***Activité de l'eau Aw.*** L'activité de l'eau (également dénommée « activité de l'eau libre ») est une mesure du statut énergétique de l'eau dans un système, et, de plus, est un bon indicateur de la dégradation des matières périssables. Plus simplement, l'Aw indique la disponibilité de l'eau d'un produit pour des réactions chimiques, biochimiques, un changement d'état ou un développement de microorganismes. L'Aw représente littéralement le rapport de pression de vapeur d'eau d'un produit humide sur la pression de vapeur d'eau pure à la même température. Les valeurs d'Aw sont comprises entre 0 et 1, 1 étant la valeur d'Aw correspondant à l'eau liquide.

***Activité critique de l'eau Aw.*** L'Aw critique d'un produit est la valeur d'Aw déterminée à partir de l'isotherme de sorption d'un produit donné pour laquelle un changement d'état de la matière apparaît (prise en masse ou transition vitreuse).

***Teneur en eau.*** La teneur en eau (également dénommée « teneur en humidité ») d'un produit est la quantité d'eau (en pourcentage massique) du produit quel que soit son état dans le produit. Elle est à distinguer de l'Aw.

***Isothermes de sorption*/*désorption.*** Les isothermes de sorption/désorption sont des courbes décrivant la relation entre l'Aw et la teneur en eau d'un produit à une température constante, plus précisément les isothermes décrivent les phénomènes d'adsorption et de désorption de l'eau pour un produit donné. Une isotherme est propre à chaque produit à une température donnée. D'un point de vue structurel, les phénomènes d'adsorption et de désorption sont très fortement dépendants de la taille des particules, de leur état amorphe et de leur surface spécifique.

### Brève description des dessins

Certains aspects de l'invention seront mieux appréhendés à la lecture de la description détaillée présentée ci-après, faite en référence aux figures dans lesquelles :
- la figure 1 présente le graphique radar de la cellulose microcristalline 102, testée en tant qu'excipient de référence dans l'exemple 1,
- les figures 2A-2F présentent les graphiques radar des six excipients aux fins de l'invention testés dans l'exemple 1,
- les figures 3 et 4 sont des graphiques présentant le suivi de la teneur en humidité dans le temps en enceinte climatique à 40°C/75%HR des susdits excipients aux fins de l'invention, testés dans l'exemple 1,
- les figures 5 et 6 sont des graphiques présentant le suivi de l'activité de l'eau (Aw) dans le temps en enceinte climatique à 40°C/75%HR des six excipients aux fins de l'invention, testés dans l'exemple 1,
- les figures 7 et 8 présentent les isothermes de sorption des six excipients aux fins de l'invention testés dans l'exemple 1,
- la figure 9 présente la superposition du graphique radar du produit de référence testé à l'exemple 2 (Arkogélule^{®} Gelée Royale) et du graphique radar du produit reformulé selon l'invention (obtenu à l'exemple 2),
- la figure 10 présente le graphique radar du produit selon l'invention obtenu à l'exemple 3,
- les figures 11 et 12 correspondent respectivement aux courbes de suivi d'évolution de l'activité de l'eau libre dans le temps et de la teneur en eau en enceinte climatique à 40°C/75%HR dans le temps de la composition selon l'invention, obtenue à l'exemple 3,
- la figure 13 présente le graphique radar correspondant à la composition selon l'invention, obtenue à l'exemple 4,
- les figures 14 et 15 correspondent respectivement aux courbes de suivi d'évolution de l'activité de l'eau libre dans le temps et de la teneur en eau en enceinte climatique à 40°C/75%HR dans le temps de la composition selon l'invention, obtenue à l'exemple 4,
- les figures 16 et 17 correspondent respectivement aux graphiques radar obtenus à partir de la poudre de ginseng bio et de l'extrait de ginseng bio présents dans la composition de l'exemple 5,
- les figures 18 et 19 correspondent respectivement aux graphiques radar obtenus à partir de l'extrait sec de thé vert bio et de la poudre de piloselle bio présents dans la composition de l'exemple 6, et
- la figure 20 présente le graphique radar obtenu à partir de la susdite composition de l'exemple 6.

### Description détaillée de l'invention

La description détaillée ci-après a pour but d'exposer l'invention de manière suffisamment claire et complète, notamment à l'aide d'exemples, mais ne doit en aucun cas être regardée comme limitant l'étendue de la protection aux modes de réalisation particuliers et aux exemples présentés ci-après.

### Exemples

### Exemple 1 : Evaluation et performances pharmaco-techniques d'excipients aux fins de l'invention et comparaison avec un excipient de référence et des excipients dits « comparatifs » (à savoir ne répondant pas à la définition de l'invention)

### 1.1. Excipients testés

### 1.1.1. Excipients aux fins de l'invention

A titre d'excipients aux fins de l'invention ont été testés les produits suivants :
i) Fibres de pomme bio VITACEL^{®} grade AF400-30 POMME BIO EC009986 (J. RETTENMAIER & SOHNE (JRS), longueur moyenne des fibres env. 30 µm ; sous forme de particules micronisées),
ii) Fibres d'avoine BIO EC010030 ORGANIC OAT FIBRE M20 (GREENFIELD).
iii) Fibres de sarrasin bio EC010742 ORGANIC BUCKWHEAT FIBRE M20 (GREENFIELD distribué par ESENCO)
iv) Fibres d'avoine EC011037 OAT FIBRE M20 (GREENFIELD distribué par ESENCO)
v) Fibres d'aronia EC010872 ARONIA FIBRE M20 (GREENFIELD distribué par ESENCO)
vi) Fibres de cassis EC0011038 CASSIS FIBRE M20 (GREENFIELD distribué par ESENCO)

Les paramètres « teneur en fibres totales », « teneur en fibres solubles dans l'eau », « teneur en fibres insolubles dans l'eau » et « humidité maximum » (« humidité max » ; selon le fournisseur) des excipients aux fins de l'invention sont résumés dans le tableau 1 infra.

**Tableau 1**

| Excipient aux fins de l'invention | Teneur en fibres totales | Dont teneur en fibres solubles dans l'eau | Dont Teneur en fibres insolubles dans l'eau | Humidité max. selon le fournisseur |
|---|---|---|---|---|
| Fibres de pomme bio | 60% | 15% | 45% | ≤ à 8.0% |
| Fibres d'avoine bio M20 | Min 60% | 2% | 58% | ≤ à 8.0% |
| Fibres aronia M20 | ≈ 67% | 5% | 62% | ≤ à 7.0% |
| Fibres de sarrasin bio M20 | Min 85% | 0.085% | 76.5% | ≤ à 7.0% |
| Fibres avoine M20 | Min 64% | 2% | 62% | ≤ à 8.0% |
| Fibres de cassis M20 | Min 60% | 7% | 53% | ≤ à 7.0% |
| Fibres de pois | Min 70% | 0% | 70% | ≤ à 10.0% |

### 1.1.2. Excipient de référence

La cellulose microcristalline 102 (n° CAS : 9004-34-6) a été testée en tant qu'excipient de référence.

Pour rappel, la cellulose est un polymère naturel de D-glucose, dans lequel la liaison entre les unités de glucose est de type β, 1→4. La cellulose microcristalline est principalement issue de la fibre végétale, présente comme composante de la paroi cellulaire sous forme de faisceaux de microfibrilles. Ces microfibrilles sont composées à la fois de cellulose amorphe et de cellulose cristalline, d'hémicellulose et de lignine. La cellulose cristalline est isolée en la séparant de la lignine et de l'hémicellulose, puis de la cellulose amorphe par hydrolyse acide.

Dans l'industrie pharmaceutique, la cellulose microcristalline est principalement utilisée comme excipient en tant que diluant pour comprimés et gélules, désintégrant et agent anti-agglomérant.

Cette cellulose microcristalline est notamment utilisée en tant qu'excipient dans le produit commercialisé par la demanderesse sous la dénomination Arkogélule^{®} Gelée royale (cf. exemple 2 infra), à une quantité supérieure à 80,3% en masse par rapport à la masse totale du produit.

### 1.1.3. Excipients comparatifs ne répondant pas à la définition de l'invention

A titre d'excipients comparatifs ne répondant pas à la définition de la présente invention ont été testés les produits suivants :
- *Inuline* INULINE FRUTAFIT IQ (UNIVAR)
   L'inuline est constituée de fructo-oligosaccharide, ingrédient naturel extrait de la racine de chicorée. L'inuline est une fibre alimentaire végétale soluble.
- *Gomme d'acacia* bio FIBREGUM^{™} BIO (NEXIRA)
   La gomme d'acacia, également appelée gomme arabique, est une fibre alimentaire soluble issue de la gomme exsudée s'écoulant naturellement ou obtenue par incision du tronc ou des branches de l'*Acacia senegal* et de l'*Acacia seyal.* Elle contient environ 90% de fibres solubles.

### 1.2 Méthodologie

### 1.2.1 Construction de « graphiques radar »

Les propriétés pharmaco-techniques des excipients aux fins de l'invention testés et de l'excipient de référence (cf. point 1.1.1 supra) sont évaluées au moyen d'une démarche issue des ICH Q8, introduisant le concept de « Quality by Design » et d'une approche adaptée de la méthodologie SeDeM (cf. J. M. Sune Negre, et al. [1] et [2]), mise au point pour la préformulation et formulation de médicaments selon un procédé de compression directe. Après identification des paramètres pharmaco-techniques critiques qui sont fonction de la forme galénique à développer, cette méthode permet la construction de graphiques dits « graphiques radar » (également dénommés « profils radar »).

Les résultats pondérant les propriétés pharmaco-techniques identifiées comme d'intérêt, sont ainsi présentés sous cette forme dite « graphique radar » afin d'être facilement, d'une part, comparés entre eux, et d'autre part, comparés avec ceux d'une ou plusieurs substances pulvérulentes pouvant être différentes. Grâce à la reproductibilité de la méthode, les avantages et inconvénients de chaque substance pulvérulente à mettre en oeuvre sont ainsi facilement identifiés, et les choix de formulation justifiés. De plus, un autre avantage non négligeable de cette méthode, est qu'elle est facilement adaptable sur la base de la connaissance des contraintes industrielles de la demanderesse. Cette démarche permet de pondérer la robustesse des formulations dès les phases de recherche et de développement, bien en amont de la transposition industrielle.

Les paramètres critiques identifiés pour la mise en forme gélule sont les suivants :
a. Masse volumique (Da) exprimée en g/ml, qui est un facteur d'incidence de type dimensionnel et dont la méthode est définie dans la monographie de la Pharmacopée européenne (version en vigueur P.E.2.9.34),
b. Masse volumique tassée (Dc) exprimée en g/ml, facteur d'incidence de type dimensionnel dont la méthode de contrôle est également définie par la monographie PE 2.9.34, le volume considéré est la valeur lue après 2500 chocs ;
c. Indice de Carr (IC) ou indice de compressibilité, facteur d'incidence relatif à la compressibilité obtenu par calcul, dont la formule est définie dans la monographie de la Pharmacopée européenne (version en vigueur P.E.2.9.34) ;
d. Indice d'Hausner (IH), facteur d'incidence relatif à l'écoulement et la fluidité obtenu par calcul, dont la formule est définie dans la monographie de la Pharmacopée européenne (version en vigueur P.E.2.9.34) ;
e. Ecoulement de la poudre à travers un orifice (nommé sous le nom de l'appareil utilisé « Flodex^{™} »), facteur d'incidence relatif à l'écoulement et la fluidité, méthodologie interne adaptée à partir des prescriptions de l'USP29-1174 POWDER FLOW et coulabilité des poudres par la méthode de coulabilité intrinsèque des poudres A. GIOIA (cf. [3] ; Flodex™, HANSON RESEARCH CORPORATION, USA) ;
f. Distribution granulométrique ;
g. Taux d'humidité et suivi %HR en étuve 40°C/75%HR, facteur d'incidence lubrification/stabilité, selon une méthodologie définie en interne : la poudre est placée en conditions environnementales de stress (en vrac en boite de Pétri, laquelle est placée en enceinte climatique à 40°C/75%HR), conditions qui favorisent un vieillissement accéléré. Les mesures du taux d'humidité sont effectuées à T0, T_{2H00}, T_{4H00}, T _{6H00} et T _{24H00} par thermogravimétrie au moyen d'une thermobalance infra-rouge (MA 45 Analyseur d'humidité, SARTORIUS, Allemagne). L'échantillon est réparti de manière régulière et en fine couche sur une coupelle dédiée, est pesé sur la thermobalance puis chauffé jusqu'à une température de105°C grâce aux rayons infrarouge jusqu'à arrêt automatique à masse constante ;
h. Eau libre (Aw) et suivi Aw en étuve à 40°C/75%HR, facteur d'incidence lubrification/stabilité, selon une méthodologie définie en interne. La poudre est placée en conditions environnementales de stress (en vrac en boîte de Pétri, laquelle est placée en enceinte climatique à 40°C/75%HR), conditions qui favorisent un vieillissement accéléré. Les mesures de l'activité de l'eau libre sont effectuées à T0, T_{2H00}, T_{4H00}, T _{6H00} et T _{24H00} par la technique du point de rosée au moyen d'un Aw-mètre (4TE Dew Point Water Activity Meter, AQUALAB,Decagon Devices, Inc., USA). L'échantillon de poudre est placé dans une cupule en plastique dédiée, puis dans l'Aw-mètre au niveau de l'emplacement prévu à cet effet. Il suffit de refermer le capot de la machine pour lancer l'acquisition. L'arrêt de la mesure est automatique et la valeur de mesure d'Aw directement lue sur l'affichage numérique. D'une part, un produit présentant une valeur d'Aw suffisamment modérée (< à 0,6) permet de garantir une stabilité microbiologique conforme aux spécifications de la demanderesse (selon PE 5.1.4). D'autre part, d'un point de vue physico-chimique une valeur d'Aw inférieure à la valeur d'Aw critique garantit l'intégrité de l'état physique du produit.

Concernant la méthode utilisée aux fins de la détermination du paramètre e) supra (écoulement de la poudre à travers un orifice), il convient de noter que cette méthode permet de ne prendre en compte que les frottements interparticulaires de la substance pulvérulente, et de s'affranchir de tout ou partie des frottements particules de poudre-paroi contrairement à la méthode classique d'écoulement à travers un entonnoir de type PE (2.9.16). Cet appareil est constitué, entre autres, d'un entonnoir métallique, d'un cylindre métallique servant de trémie pour la poudre, de disques plats présentant différents diamètres d'orifice en leur centre, allant de 4mm à 34mm, d'un second cylindre en plastique blanc servant de support aux disques, et d'un système de trappe fixé sur un support de type tige, et obturant l'orifice au début de la manipulation. La prise d'essai de l'échantillon de poudre à considérer est de 50g. Un disque d'un diamètre choisi est installé sur le cylindre en plastique blanc situé sous le cylindre métallique ; la trappe située sous les cylindres est refermée obturant ainsi l'orifice du disque. L'échantillon de poudre est versé dans le cylindre métallique par le biais de l'entonnoir ; puis la trappe est ouverte et on pèse la masse de poudre écoulée. Le but est de continuer ainsi, en augmentant ou en diminuant le diamètre de l'orifice du disque, jusqu'à obtenir une masse écoulée supérieure ou égale à 30% de la masse de poudre au départ. La valeur du diamètre du disque correspondant est considérée comme le « seuil de rupture ». Sur la base des connaissances de la demanderesse rapportées à son outillage industriel, il a été défini que le disque de diamètre 16 mm correspond au seuil de rupture idéal pour ses poudres. Un seuil critique d'écoulement obtenu pour des diamètres d'orifice supérieurs à 24mm a été considéré comme non satisfaisant. En dessous de 16mm, l'écoulement est considéré comme bon à excellent. L'objectif de cette méthode est de qualifier non pas la vitesse d'écoulement ni le débit, mais le diamètre d'ouverture minimum nécessaire à un écoulement de la poudre en masse.

Concernant le paramètre pharmaco-technique f supra (Distribution granulométrique), celui-ci est traditionnellement exprimé comme la distribution granulométrique par tamisage (% P>à 90µm), facteur d'incidence lubrification/dosage, dont la méthode de contrôle est également définie par la monographie PE 2.9.38, la colonne de tamis utilisée est composée des tamis 710µm, 500µm, 355µm, 250µm, 180µm, 125µm, 90µm et fond de tamis. Toutefois, et contrairement aux attentes originelles de la demanderesse, les résultats exprimant la distribution granulométrique par tamisage (% P>à 90µm), facteur d'incidence lubrification/dosage, n'ont pu figurer sur le graphique radar, car la méthode de contrôle PE 2.9.38 s'avère ne pas être appropriée au vu de la finesse des particules des fibres alimentaires végétales d'intérêt. Il a été observé que les particules ont un comportement collectif entraînant une tendance au colmatage des grilles de tamis, faussant ainsi les résultats. L'ensemble des fibres d'intérêts ont alors été analysées par granulométrie laser qui est une méthode de détermination plus précise et plus spécifique pour l'analyse des particules de taille inférieure à 90 µm. Les résultats de cette analyse granulométrique sont présentés au point 1.3.2 infra.

En résumé, la construction pour chaque excipient aux fins de l'invention d'un graphique dit « graphique radar » (également dénommé « profil radar ») permet une évaluation rapide des avantages et des limites de ces excipients en termes de propriétés pharmaco-techniques et, le cas échéant, également une comparaison rapide des différents profils entre eux. Plus précisément, la lecture des graphiques « radar » présentés au sein de la présente demande de brevet doit être réalisée comme suit : la cible attendue pour chacun des critères testés doit être au minimum de 5 (sur 10) pour être considérée comme un critère performant, sauf en ce qui concerne le pourcentage d'humidité relative (%HR) et l'activité de l'eau libre (Aw) pour lesquels la lecture est inversée. En effet, pour chacun de ces deux critères, la valeur cible attendue doit être idéalement la plus proche possible de 0 (sur 10) ; 5 (sur 10) étant un résultat moyen et au-delà, la performance pour ces deux critères sera considérée de médiocre à critique. Le pourcentage d'humidité relative (%HR) et l'activité de l'eau libre (Aw) sont considérés comme des critères clés au sens de la présente invention.

### 1.2.2 Suivi de la teneur en humidité (Δ%HR) en enceinte climatique à 40°C/75%HR

La poudre est placée en conditions environnementales de stress (en vrac en boîte de Pétri, laquelle est placée en enceinte climatique à 40°C/75%HR), conditions qui favorisent un vieillissement accéléré. Les mesures du taux d'humidité sont effectuées à T0, T_{2H00}, T_{4H00}, T _{6H00} par thermogravimétrie au moyen d'une thermobalance infra-rouge (MA 45 Analyseur d'humidité, SARTORIUS, Allemagne). L'échantillon est réparti de manière régulière et en fine couche sur une coupelle dédiée, est pesé sur la thermobalance puis chauffé jusqu'à une température de 105°C grâce aux rayons infrarouge jusqu'à arrêt automatique à masse constante.

### 1.2.3 Suivi de l'activité de l'eau en enceinte climatique à 40°C/75%HR

La poudre est placée en conditions environnementales de stress (en vrac en boîte de Pétri, laquelle est placée en enceinte climatique à 40°C/75%HR), conditions qui favorisent un vieillissement accéléré. Les mesures de l'activité de l'eau libre sont effectuées à T0, T_{2H00}, T_{4H00}, T _{6H00} par la technique du point de rosée au moyen d'un Aw-mètre (4TE Dew Point Water Activity Meter, AQUALAB,Decagon Devices, Inc., USA). L'échantillon de poudre est placé dans une cupule en plastique dédiée, puis dans l'Aw-mètre au niveau de l'emplacement prévu à cet effet. Il suffit de refermer le capot de la machine pour lancer l'acquisition. L'arrêt de la mesure est automatique et la valeur de mesure d'Aw directement lue sur l'affichage numérique.

### 1.2.4 Etablissement d'isothermes de sorption

Par ailleurs, les isothermes de sorption des excipients aux fins de l'invention (listés au point 1.1.1 supra) ont été réalisées au moyen d'un analyseur de sorption de vapeur AquaLab (VSA, AQUALAB,Decagon Devices, Inc., USA), afin de déterminer leur capacité d'absorption en eau, et d'identifier leur éventuel changement d'état en cas de dépassement d'Aw critique.

De même, les isothermes de sorption de l'excipient de référence (cf. point 1.1.2 supra) et des excipients comparatifs (cf. point 1.1.3 supra) ont été réalisés afin de pouvoir justifier des conclusions apportées sur les résultats obtenus sur les excipients aux fins de l'invention. Les résultats sont présentés au point 1.3.5 infra.

### 1.3 Résultats

### 1.3.1 Profils radar

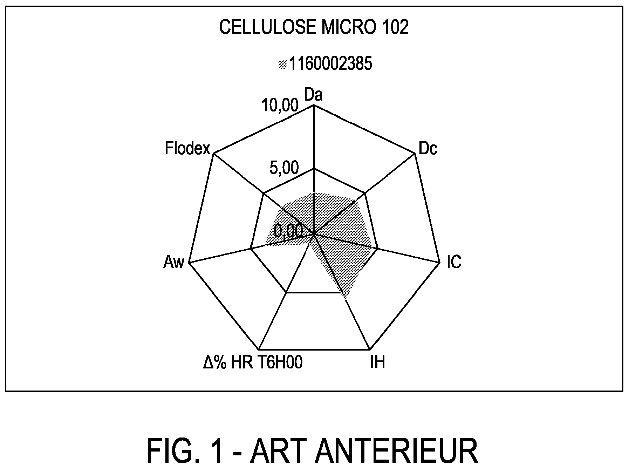

Le profil radar de référence, obtenu pour l'excipient de référence (cellulose microcristalline 102) en mettant en oeuvre la méthodologie définie au point 1.2. supra est présenté en figure 1.

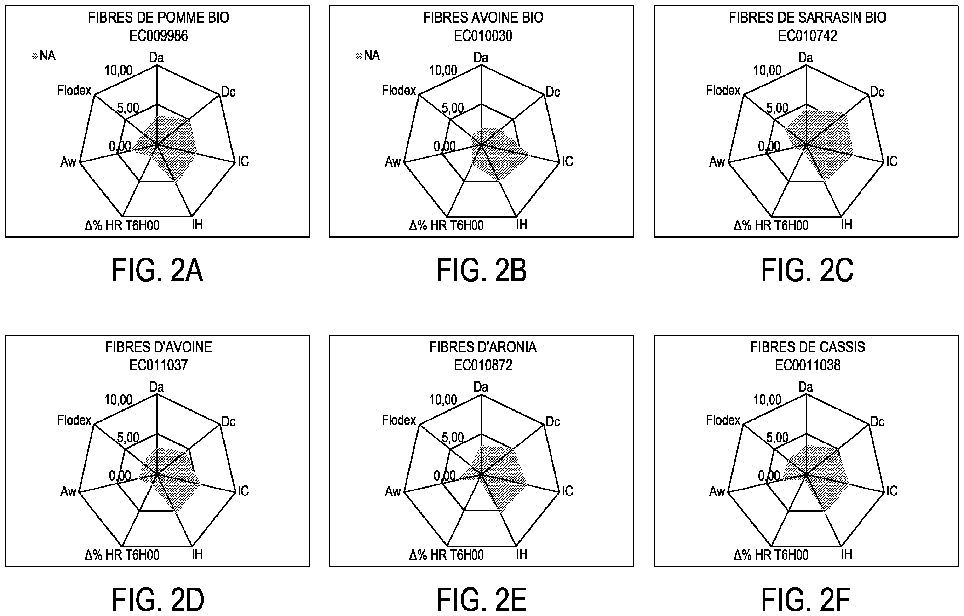

Les profils radar obtenus en mettant en oeuvre la susdite méthodologie pour les excipients aux fins de l'invention sont présentés en figures 2A (VITACEL^{®} fibres de pomme BIO EC009986), 2B (fibres d'avoine BIO EC0010030), 2C (fibres de sarrasin bio EC010742 ORGANIC BUCKWHEAT FIBRE M20), 2D (fibres d'avoine EC011037 OAT FIBRE M20 (GREENFIELD distribué par ESENCO), 2E (fibres d'aronia EC010872 ARONIA FIBRE M20) et 2F (fibres de cassis EC0011038 CASSIS FIBRE M20).

### 1.3.2 Résultats de l'analyse granulométrique

Les résultats de l'analyse de la distribution granulométrique par granulométrie laser sont présentés dans le tableau 2 infra.

Les excipients ont été classés en fonction des résultats d'analyse par ordre croissant de granulométrie.

**Tableau 2**

| Excipient aux fins de l'invention | Distribution en taille des particules Dv10 | Distribution en taille des particules Dv50 | Distribution en taille des particules Dv90 |
|---|---|---|---|
| Fibres de pomme bio EC009986 | 3,89 µm | 17,5 µm | 41,2 µm |
| Fibres d'avoine bio M20 EC0010030 | 2,52µm | 14,6 µm | 225 µm |
| Fibres aronia M20 EC010872 | 7,44 µm | 32,6 µm | 129 µm |
| Fibres de sarrasin bio M20 EC010742 | 4,59 µm | 48,4 µm | 160 µm |
| Fibres avoine M20 EC011037 | 5,43µm | 46,3 µm | 188 µm |
| Fibre de cassis M20 EC0011038 | 14,7 µm | 65 µm | 173µm |
| Fibres de pois EC011557 | 0,87 µm | 4,3 µm | 8 µm |

### 1.3.3 Suivi de la teneur en humidité (Δ%HR) en enceinte climatique à 40°C/75%HR

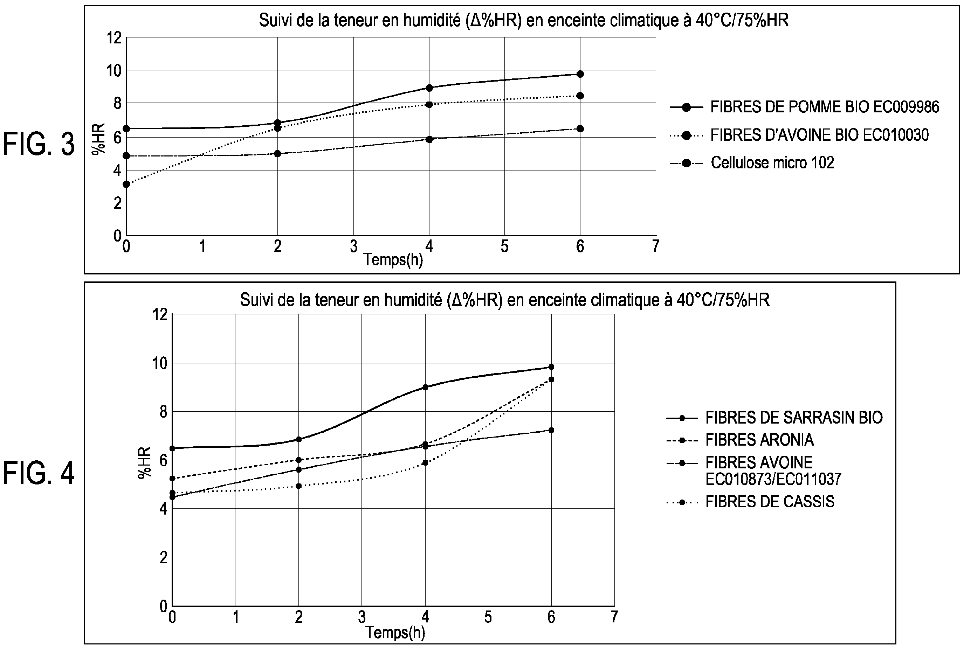

Le suivi de la teneur en humidité en enceinte climatique à 40°C/75%HR (humidité relative) a été effectué pour chacun des six excipients aux fins de l'invention présentés au point 1.1.1, ainsi que pour l'excipient de référence. Les résultats sont présentés sous forme de graphique en figures 3 et 4.

### 1.3.4 Suivi de l'activité de l'eau en enceinte climatique à 40°C/75%HR

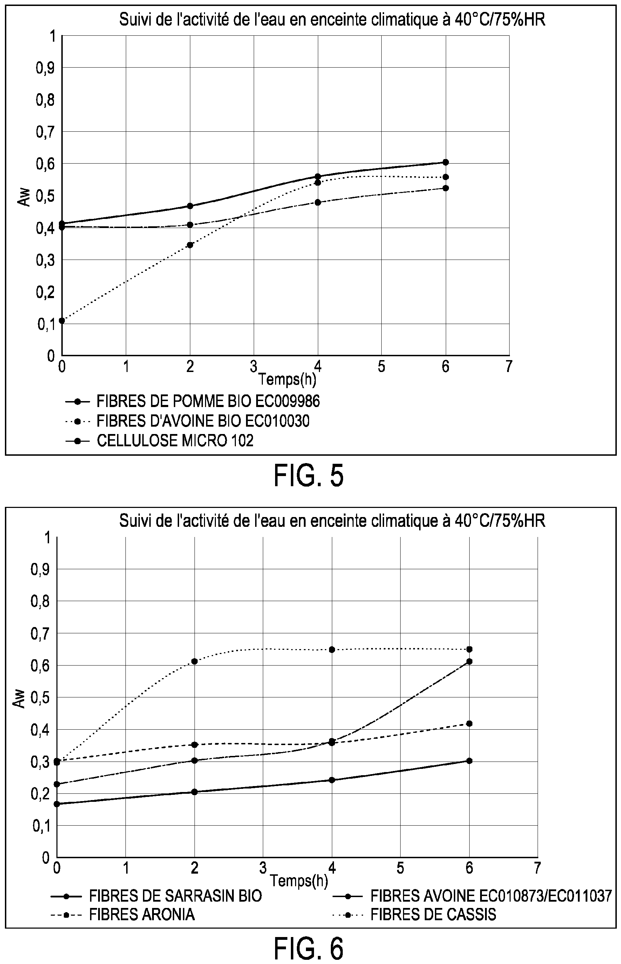

Le suivi de de l'activité de l'eau en enceinte climatique à 40°C/75%HR (humidité relative) a été effectué pour chacun des six excipients aux fins de l'invention présentés au point 1.1.1, ainsi que pour l'excipient de référence. Les résultats sont présentés sous forme de graphique en figures 5 et 6.

### 1.3.5 Isothermes de sorption

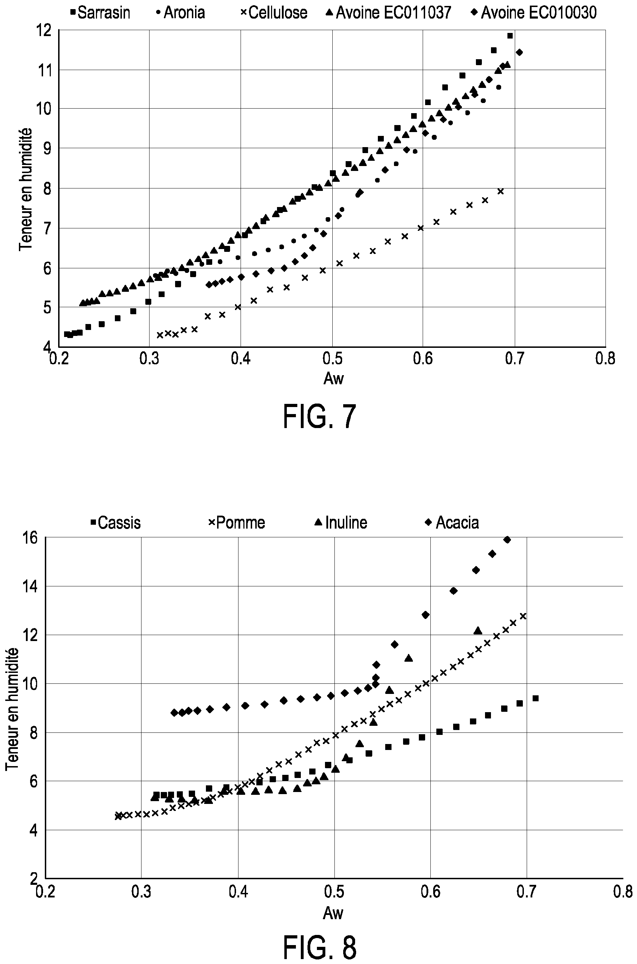

Les isothermes de sorption réalisées à partir des excipients aux fins de l'invention, de l'excipient de référence et des excipients comparatifs sont présentées en figures 7 et 8. En outre, et dans un souci d'exhaustivité, les observations réalisées en lien avec ces isothermes de sorption sont résumées au sein du tableau 3 infra.

**Tableau 3**

| Excipient testé | Δ %HRE | Observations |
|---|---|---|
| CELLULOSE MICRO 102 | T0= 4.3% | En fin de test la poudre a légèrement motté. |
| | Tf = 8.2% | |
| | Soit un Δ %HRE = 3.9% | |
| GOMME ACACIA | T0= 9% | A 25°C et à 30°C =>mottage se produit à partir d'une humidité ambiante de 52% environ et pour une teneur en eau de la poudre voisine de 10%. |
| | Tf = 16% | |
| | Soit un Δ %HRE = 7% | |
| INULINE (non BIO) | T0= 5.2% | A 25°C, deux points d'inflexion sont relevés témoignant de deux changements d'état successifs. |
| | Tf = 12.8% | |
| | Soit un Δ %HRE = 7.6% | |
| CASSIS FIBRES EC011038 | T0= 5.4% | Poudre inchangée à 25°c et à 30°C pour une humidité ambiante pouvant évoluer jusqu'à 70%HR |
| | Tf = 9.4% | |
| | Soit un Δ %HRE = 4.0% | |
| FIBRES POMME BIO EC009986 | T0= 5.6% | Pas de phénomènes de mottage ni de prise en masse. La poudre à tendance à adhérer un peu sur les surfaces dans une ambiance humide voisine de 70%HR. |
| | Tf = 11.4% | |
| | Soit un Δ %HRE = 5.8% | |
| FIBRES AVOINE code EC011037 | T0= 5.6% | Pas de changement d'aspect de la poudre |
| | Tf = 11.3% | |
| | Soit un Δ %HRE = 5.7% | |
| FIBRES SARRASIN BIO EC010742 | Δ %HRE à 25°C = 12-4= 8%HRE | Forte capacité d'absorption de l'eau. A une humidité voisine de 45%HR, un léger mottage apparait mais est très facilement dispersible. |
| ARONIA FIBRES non BIO EC010872 | T0= 5.8 % | Pas de changement d'aspect de la poudre |
| | Tf= 11.0 % | |
| | Soit un Δ %HRE = 5.2% | |
| FIBRES AVOINE Code EC010030 | T0= 5.6% | Pas de changement d'aspect de la poudre |
| | Tf = 11.4% | |
| | Soit un Δ %HRE = 5.8% | |
| FIBRES POIS EC011557 | T0= 5% | Un léger mottage apparait mais est très facilement dispersible. La poudre a tendance à adhérer légèrement sur les surfaces dans une ambiance humide voisine de 80%HR mais cela reste acceptable. |
| | Tf = 17% | |
| | Soit un Δ %HRE = 12% | |

### 1.4. Discussion et conclusion

L'ensemble des excipients aux fins de l'invention présentent des propriétés intéressantes en termes de capacité d'absorption d'humidité. Ces propriétés, corrélées à l'absence de prise en masse, et ce même après une exposition prolongée en conditions drastiques d'humidité et de température (enceinte climatique 40°C/75%HR), permettent de conclure que les susdits excipients aux fins de l'invention représentent des agents anti-agglomérants (en en particulier des agents protecteurs contre l'humidité) de première importance notamment lorsque l'on utilise des matières premières hygroscopiques (telles que certaines poudres de plante(s) et la quasi-totalité des extraits secs de plantes), qui plus est, conformes aux réglementations idoines en matière de produits biologiques et notamment au règlement CE n° 889/2008 du 5 septembre 2008 (et ses modifications).

Même si les propriétés d'écoulement intrinsèques des excipients aux fins de l'invention testés semblent être inférieures à celles obtenues à partir de l'excipient de référence (cellulose microcristalline 102), ces propriétés pourront, le cas échéant, être compensées qualitativement et quantitativement par l'ajout d'un ou plusieurs autres agent(s) de charge approprié(s).

Au cours des essais de suivi de la teneur en eau et activité de l'eau réalisés, il a été constaté qu'aucune des fibres alimentaires végétales aux fins de l'invention ne présentait d'aptitude à la prise en masse ni de changement organoleptique majeur malgré une capacité d'absorption d'humidité importante mise en évidence. Cette aptitude est précisément une des propriétés clés recherchées pour la protection de substances actives/ingrédients actifs sensibles pour lesquels les fibres végétales testées joueront le rôle de « tampon » absorbeur d'humidité et d'anti-agglomérant.

Les données en la matière figurant sur le graphique radar, ont été complétées par la construction de graphiques comparatifs de l'évolution :
- de la teneur en humidité (%HR) de l'échantillon (à 40°C/75%HR)
- de l'activité de l'eau de l'échantillon (à 40°C/75%HR)
Les graphiques permettent une comparaison plus facile visuellement des fibres d'intérêt entre elles, et notamment en ce qui concerne l'évolution de la teneur en humidité (%HR), et la mise en évidence d'une évolution similaire pour l'ensemble des fibres d'intérêt étudiées ainsi que pour l'excipient de référence. Cela se traduit notamment par des valeurs initiales de teneur en eau basses (< à 7% HR), suivi d'une augmentation importante de l'humidité au bout de 6H00 (six heures) en enceinte climatique à 40°C/75%HR. Ces résultats sont d'autant plus évocateurs qu'ils sont à corréler à l'absence de modification des caractéristiques organoleptiques et de pulvérulence de chacune des fibres alimentaires végétales d'intérêt.

Réalisé en parallèle, le suivi de l'activité de l'eau a également permis de dégager une tendance d'évolution de cette caractéristique de façon très macroscopique comparativement à la réalisation des isothermes de sorption, mais somme toute intéressante, notamment grâce aux valeurs initiales d'Aw basses, toutes inférieures ou égales à 0,4.

En outre, les isothermes de sorption obtenus démontrent, pour l'ensemble des fibres d'intérêt, une absence de dépassement d'Aw critique c'est-à-dire une absence de prise en masse ou de changement d'état de type vitrification pour des valeurs d'Aw élevées, c'est-à-dire supérieures à 0,7. Ces observations sont à opposer aux résultats obtenus sur les excipients comparatifs et l'excipient de référence, pour lesquels une ou plusieurs valeurs d'Aw critique(s) a/ont été détectée(s) révélant au mieux une prise en masse potentiellement réversible, dans le cas de l'excipient de référence, au pire une prise en masse irréversible voire une vitrification, dans le cas de l'ensemble des excipients comparatifs.

En outre, à partir des isothermes de sorption, la demanderesse est parvenue à déterminer que l'ensemble des fibres aux fins de l'invention présentent des teneurs en eau et valeur d'Aw de départ basses (< à 8% pour la teneur en eau et < 0,5 pour la valeur d'Aw), et une capacité d'absorption en eau importante en quantité et de façon progressive dans le temps, ce qui tend à démontrer que l'eau est bien absorbée par les fibres d'intérêt tout en maintenant un équilibre dans l'environnement immédiat de la composition. Une teneur en eau inférieure à 8% et/ou une valeur d'Aw inférieure à 0,5 est/sont des paramètres préférentiels au sens de la présente invention.

### Exemple 2 : Reformulation d'un produit de référence : Arkoqéiuie^{®} Gelée royale

### 2.1. Composition du produit de référence (Arkogélule^{®} Gelée royale)

**Tableau 4**

| Désignation du composant | Fonction | % massique |
|---|---|---|
| GELEE ROYALE LYOPHILISEE BIO | Substance active | 18,667% |
| CELLULOSE MICROCRISTALLINE 102 | Excipient (agent de charge et anti-agglomérant/stabilisant) | 80,333% |
| STEARATE DE MAGNESIUM | anti-agglomérant | 1,000% |

### 2.2. Composition du produit reformulé (selon l'invention)

Le produit reformulé conformément à la présente invention présente la composition suivante :

**Tableau 5**

| Désignation du composant | Fonction | % massique |
|---|---|---|
| GELEE ROYALE LYOPHILISEE BIO | Substance active | 18,667% |
| FIBRES POMME BIO EC009986(JRS) | Excipient (agent de charge et anti-agglomérant/stabilisant) | 18,667% |
| GOMME ACACIA BIO (FIBREGUM BIO NEXIRA) | Excipient (agent de charge ; agent permettant d'améliorer les propriétés d'écoulement) | 62,6667% |

La mise en oeuvre d'une quantité de fibres alimentaires végétales FIBRES POMME BIO équivalente en poids pour poids à celle de la substance active a permis de protéger cette dernière avec une efficacité suffisante pour éviter le phénomène de prise en masse et de changement de propriétés organoleptiques.

En outre, le ratio substance active/ FIBRES POMME BIO a permis la mise en oeuvre d'une quantité suffisante de GOMME ACACIA BIO afin d'améliorer efficacement les propriétés d'écoulement de la composition selon l'invention.

### 2.3. Superposition des profils radar

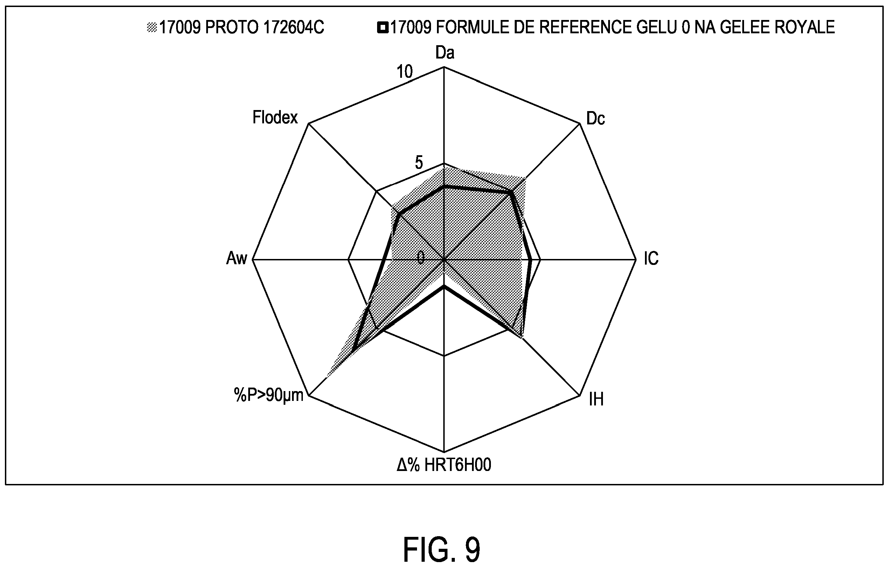

Les profils radar du produit de référence et du produit reformulé selon l'invention ont été obtenus en mettant en oeuvre la méthodologie décrite au point 1.2. supra. A des fins de comparaison, la figure 9 présente une superposition des deux profils radar ainsi obtenus. Comme illustré en figure 9, la composition selon l'invention (100% végétale et conforme aux réglementations idoines en matière de produits biologiques et notamment au règlement CE n° 889/2008 du 5 septembre 2008 et ses modifications) possède des propriétés pharmaco-techniques (et donc un comportement) au moins équivalentes voire supérieures au produit de référence (Arkogélule^{®} Gelée royale), dont le comportement sur machine à l'échelle industrielle est bien connu et satisfaisant.

### 2.4. Etude de stabilité (en étuve à 40°C/75%HR)

Une étude de stabilité a été réalisée pour le produit de référence et pour le produit reformulé selon l'invention en étuve à 40°C (75%HR) durant 45 jours.

Il ressort de cette étude de stabilité que les propriétés organoleptiques restent identiques entre le produit reformulé selon l'invention et le produit de référence. Aucune évolution en termes de prise en masse, de changement de couleur ou d'aspect de la poudre n'est relevée (par rapport au T0 à température ambiante).

### Exemple 3 : Préparation d'un produit selon l'invention : Arkogélule^{®} propolis BIO

### 3.1. Inconvénients relevés lors de l'utilisation d'un extrait sec de propolis BIO

Après essais, des inconvénients majeurs liés à l'emploi d'un extrait sec de propolis BIO se révèlent être la prise en masse et l'excédent d'humidité environnante. Il convient donc de mettre au point une composition protégeant l'extrait sec de propolis BIO de la prise en masse et capable d'absorber l'excédent d'humidité environnante, tout en étant conforme aux réglementations idoines en matière de produits biologiques et notamment au règlement CE n° 889/2008 du 5 septembre 2008 et ses modifications.

### 3.2. Composition du produit selon l'invention

Confrontée à la problématique susvisée, la demanderesse a mis au point la composition présentée dans le tableau 6 infra :

**Tableau 6**

| Désignation du composant | Fonction | % massique |
|---|---|---|
| EXT SEC PROPOLIS BIO | Substance active | 62,5% |
| FIBRES POMME BIO | Excipient (agent de charge et anti-agglomérant/stabilisant) | 30,75% |
| FIBRES AVOINE BIO | Excipient (agent de charge et anti-agglomérant/stabilisant) | 6,25% |

### 3.3 Profil radar obtenu

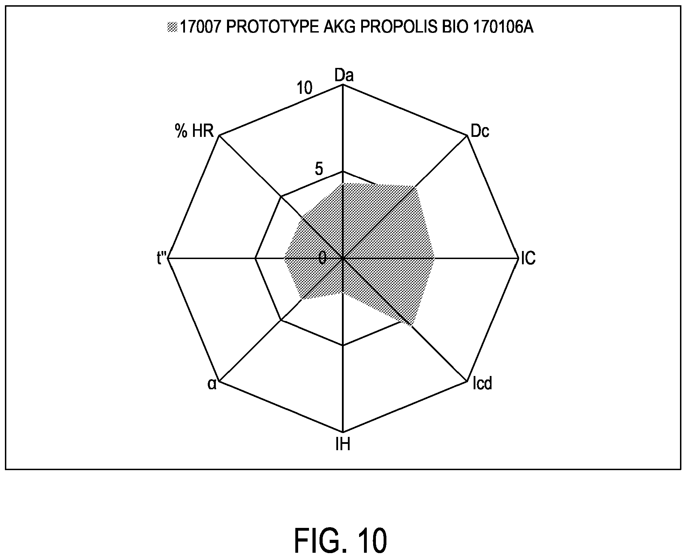

Le profil radar obtenu en mettant en oeuvre la méthodologie détaillée au point 1.2 supra est présenté en figure 10.

Comme nous pouvons le constater d'après cette figure 10, l'emploi de fibres alimentaires végétales aux fins de l'invention au sein de la composition Arkogélule^{®} propolis BIO permet d'obtenir de très bonnes propriétés pharmaco-techniques.

### 3.4. Etude de stabilité

### 3.4.1. Suivi de l'évolution de la stabilité de la composition selon l'invention en enceinte climatique à 40°C/75%HR à T30 jours

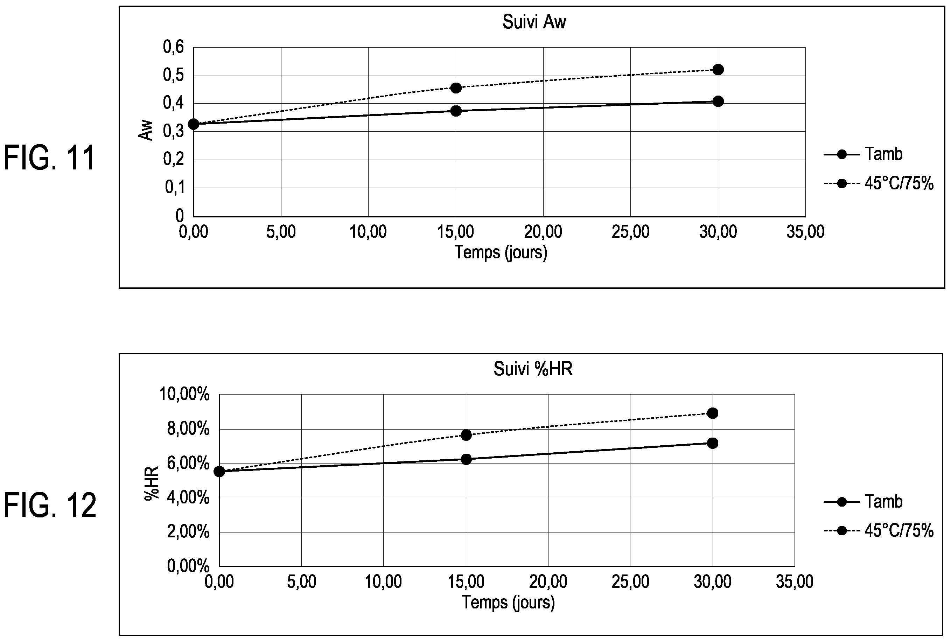

Les courbes de suivi d'évolution de l'activité de l'eau libre et de la teneur en eau en enceinte climatique à 40°C/75%HR sont respectivement présentées en figures 11 et 12.

### 3.4.2. Evaluation d'éventuelles modifications des propriétés organoleptiques

Le suivi de stabilité a été poussé à 45 jours en enceinte climatique à 40°C/75%HR. Il en ressort que les propriétés organoleptiques de la composition selon l'invention restent identiques, sans évolution de prise en masse, de changement de couleur ou encore d'aspect de la poudre (par rapport au T0 à température ambiante).

### Exemple 4 : Préparation d'un produit selon l'invention : Arkoqélule^{®} Ginkgo biloba BIO

### 4.1. Inconvénients relevés lors de l'utilisation d'un extrait sec de Ginkgo biloba

Tous les essais préalables de caractérisation ont mis en évidence le caractère hygroscopique de l'extrait sec de Ginkgo biloba, notamment par observation d'une prise en masse avec vitrification au bout de 24 heures en enceinte climatique à 40°C/75%HR.

La demanderesse a découvert que le prémélange constitué par l'association de la poudre de Ginkgo biloba feuille associée à l'extrait sec de Ginkgo biloba permettait de stabiliser le caractère hygroscopique de l'extrait lorsque le prémélange est soumis à des conditions drastiques de température et d'humidité (enceinte climatique 40°C/75%HR). Toutefois, l'ajout d'excipients aux fins de l'invention (et notamment d'au moins un agent anti-agglomérant aux fins de l'invention) s'avère primordial - voire nécessaire - pour garantir la stabilité du produit jusqu'à sa péremption.

### 4.2. Composition du produit selon l'invention

La composition du produit selon l'invention - Arkogélule^{®} Ginkgo biloba BIO - est présentée dans le tableau 7 infra.

**Tableau 7**

| Désignation du composant | Fonction | % massique |
|---|---|---|
| PDRE GINKGO BILOBA FE BIO | Substance active | 32,759% |
| EXT SEC GINKGO BILOBA BIO | Substance active | 32,759% |
| FIBRE POMME BIO | Excipient (agent de charge et anti-agglomérant/stabilisant) | 20,69% |
| CONCENTRE DE RIZ BIO | AGENT D'ECOULEMENT | 10,345% |
| GOMME ACACIA BIO | AGENT D'ECOULEMENT | 3,448% |

### 4.3 Profil radar obtenu

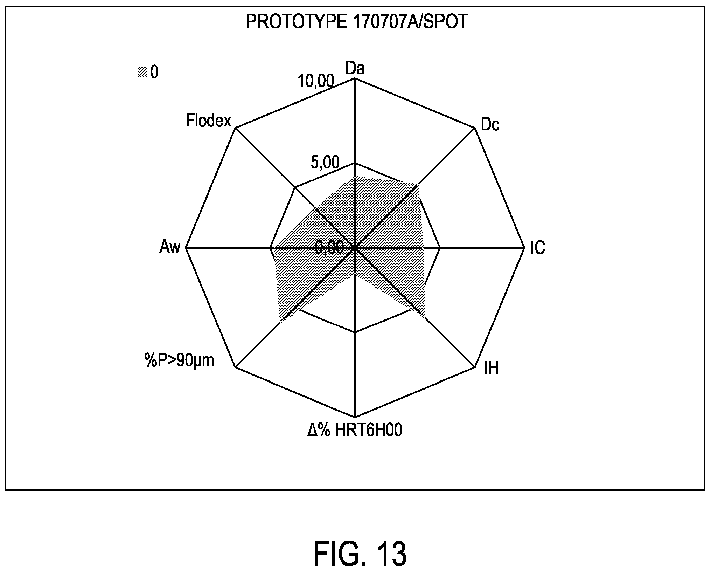

Le profil radar obtenu en mettant en oeuvre la méthodologie détaillée au point 1.2 supra est présenté en figure 13. Comme nous pouvons le constater d'après cette figure 13, l'emploi de fibres alimentaires végétales aux fins de l'invention au sein de la composition Arkogélule^{®} Ginkgo biloba BIO permet d'obtenir de très bonnes propriétés pharmaco-techniques.

### 4.4. Etude de stabilité

### 4.4.1. Suivi de l'évolution de la stabilité de la composition selon l'invention en enceinte climatique à 40°C/75%HR à T30 jours

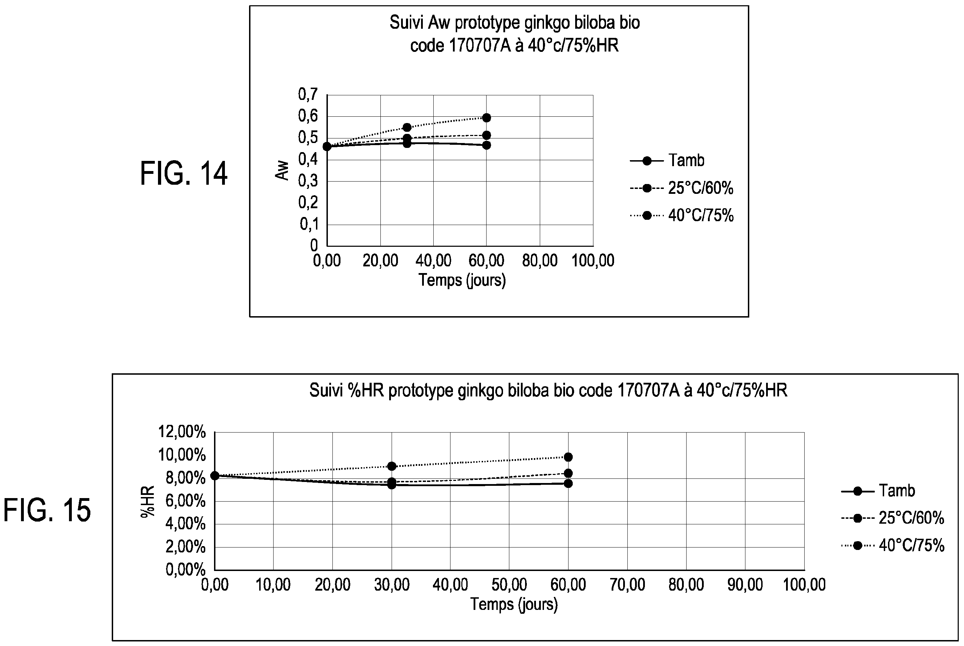

Les courbes de suivi d'évolution de l'activité de l'eau libre et de la teneur en eau en enceinte climatique à 40°C/75%HR sont respectivement présentées en figures 14 et 15.

### 4.4.2. Evaluation d'éventuelles modifications des propriétés organoleptiques

L'étude de stabilité a été poussée à deux mois en enceinte climatique à 40°C/75%HR. Il en ressort que les propriétés organoleptiques de la composition selon l'invention restent identiques par rapport à celles de la composition à T0 à température ambiante. Aucun changement de couleur ni d'aspect de la poudre n'est noté. Une légère prise en masse est observée mais reste inhérente à la formation de la carotte de poudre formée par le bourrage de la poudre par le procédé de mise en forme gélule.

### Exemple 5 : Reformulation d'un produit de référence : Arkogélule^{®} Ginseng

### 5.1. Composition du produit de référence (Arkogélule^{®} Ginseng)

**Tableau 8**

| Désignation du composant | Fonction | % massique |
|---|---|---|
| POUDRE GINSENG BIO | actif | 100% |

### 5.2 Composition du produit reformulé (selon l'invention)

Dans le cadre d'une extension de la gamme de produits Arkogélules^{®}, la demanderesse a souhaité reformuler le produit de référence (présenté au point 5.1 supra) afin de l'enrichir en extrait de la même plante. Le produit ainsi reformulé présente la composition suivante :

**Tableau 9**

| Désignation du composant | Fonction | % massique |
|---|---|---|
| POUDRE GINSENG BIO | Substance active | 50% |
| EXTRAIT SEC GINSENG BIO GRANULE | Substance active | 33.636% |
| Fibre pomme bio (JRS) EC009986 | Excipient (agent de charge et anti-agglomérant/stabilisant) | 16.364 % |

### 5.3 Observations à partir des profils radar

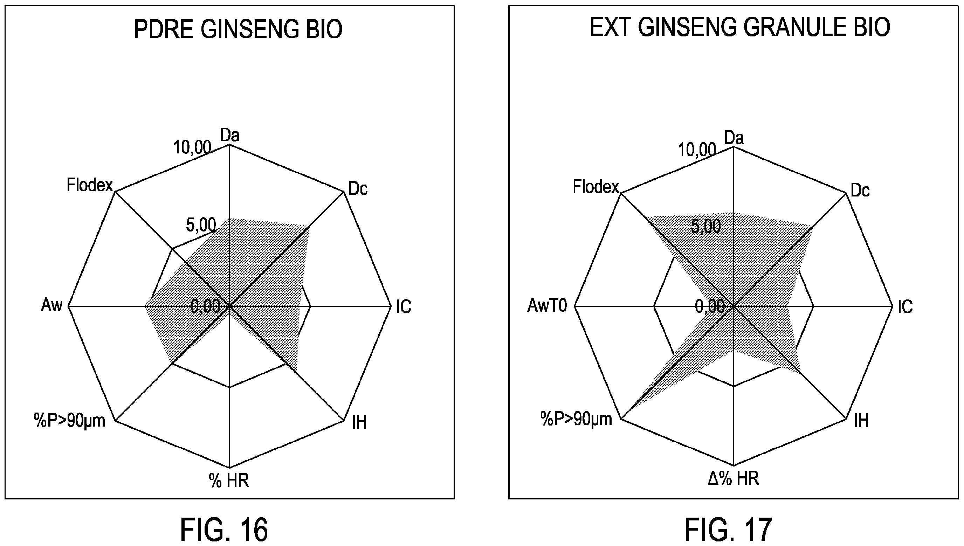

Les profils radars correspondant à la poudre de ginseng bio et à l'extrait de ginseng bio (granule) sont respectivement présentés en figures 16 et 17. Comme montré sur la figure 16, la poudre de ginseng présente un taux non négligeable de particules fines dont la taille est inférieure à 90 µm. L'écoulement de la poudre est satisfaisant car son seuil de rupture correspond au disque de diamètre 20 mm au Flodex. Concernant la stabilité, le taux d'augmentation d'humidité entre T0 et T24h est de 3,32% ce qui reste acceptable.

Concernant l'extrait de ginseng, et tel qu'illustré par la figure 17, cet extrait apparaît présenter de bonnes caractéristiques physico-chimiques. En effet, il se compose majoritairement de particules dont la taille est supérieure à 500 µm et s'avère stable au cours des 24h d'étude à 40°C/75%HR. Il présente également un écoulement plus que satisfaisant avec un seuil de rupture de 8 mm au Flodex ce qui est évidemment en lien avec son caractère granulé.

### 5.4 Problématique de déphasage et de stabilité

La différence de taille de particule étant très significative entre l'extrait de ginseng bio (granule) et la poudre de ginseng bio (500 µm vs. 90 µm), un déphasage du mélange est très largement visible.

Par ailleurs, lorsqu'un mélange poudre de ginseng bio/extrait de ginseng bio granule est placé en stabilité pour 24h à 40°C/75%HR et à 30°C/75%HR (boîte de Pétri fermée), les constatations suivantes sont effectuées : un examen visuel révèle que le susdit mélange prend légèrement en masse à la surface de la boîte de Pétri et forme des mottes. Les particules les plus grosses - correspondant à l'extrait de ginseng bio (granule) - apparaissent plus foncées qu'initialement. La couleur de l'échantillon est légèrement modifiée et devient plus foncée qu'initialement, les particules les plus fines ont tendance à motter légèrement mais restent facilement friables. Le recours à un agent anti-agglomérant (et plus précisément d'un agent protecteur contre l'humidité) apparaît donc nécessaire.

### 5.5 Apports de l'excipient aux fins de l'invention

Concernant le problème de déphasage mentionné supra, il est constaté que la fibre de pomme bio (le cas échéant en association avec un agent de charge tel que la gomme d'acacia bio (FIBREGUM BIO NEXIRA) permet de combler la différence granulométrique observée entre la poudre de ginseng bio et l'extrait de ginseng bio granule, permettant ainsi d'éviter ce phénomène de déphasage.

Concernant l'apport de l'excipient aux fins de l'invention quant à la stabilité du produit reformulé dont la composition est présentée dans le tableau 9 supra, il convient de noter que les analyses effectuées au moyen d'un analyseur de sorption de vapeur AquaLab (VSA, AQUALAB, Decagon Devices, Inc., USA), en mode « DDI », indique que la formule est stable à 25°C/60%HR et à 30°C/65%HR, sans changement d'état de la poudre et de l'aspect général des gélules contenant ladite poudre. Dans un souci d'exhaustivité, il convient de noter que le susdit mode « DDI » est un mode de mesure de la sorption dit par humidification dynamique permettant d'établir une courbe isotherme à une température prédéterminée qui trace la relation entre l'Aw de l'échantillon analysé et sa teneur en eau. L'échantillon se trouve dans la chambre de mesure étanche où il est soumis à un pourcentage d'air humide défini et continu. L'Aw et la variation de poids de l'échantillon sont mesurés pendant toute la durée de l'analyse jusqu'à équilibrage de l'Aw de l'échantillon avec l'humidité relative de l'air de la chambre.

### 5.6 Variation de la teneur en excipient aux fins de l'invention et éventuelles conséquences sur la stabilité de la composition

Les trois compositions présentées dans le tableau 10 infra ont été dérivées à partir de la composition du produit reformulé présentée au point 5.2 supra (teneur en fibre de pomme de 5%, 7% et 10% en masse, ajout de fibregum en tant qu'agent d'écoulement q.s. 440 mg) :

**Tableau 10**

| Désignation du composant | Composition A (% massique) | Composition B (% massique) | Compositions C (% massique) |
|---|---|---|---|
| PDRE GINSENG BIO | 50% | 50% | 50% |
| EXT SEC GINSENG BIO | 33.636% | 33.636% | 33.636% |
| Fibre pomme bio (JRS) | 5% | 7% | 10% |
| Gomme acacia BIO(FIBREGUM BIO NEXIRA) | 11.364% | 9.364% | 6.364% |

Ces trois compositions sont mises en stabilité à 30°C/75%HR et à 40°C/75%HR durant 24h. Un suivi de l'aspect visuel indique que, malgré une efficacité avérée de la fibre de pomme pour les trois compositions testées en termes de stabilisation de l'extrait sec de thé vert, la composition C contenant 10% de fibres de pomme présente un léger avantage en terme de pulvérulence malgré les conditions drastiques de température et d'humidité auxquelles elle est soumise. L'agent d'écoulement ajouté n'apportant qu'un avantage relatif sur l'aptitude à l'écoulement de la composition, le quantum satis sera fait sur la fibre de pomme à l'issue de la phase de développement.

### Exemple 6 : Formulation d'un nouveau produit gamme : Arkogélule^{®} Complex Minceur BIO

### 6.1. Composition du produit (selon l'invention)

Il s'agit d'une nouvelle gamme Arkogélules^{®} associant deux plantes : un extrait sec d'une plante et une poudre de plante appelée « totum ». La composition du produit selon l'invention est présentée dans le tableau 11 infra :

**Tableau 11**

| *Désignation du composant* | *Fonction* | *% massique* |
|---|---|---|
| *EXT SEC THE VERT BIO* | *Substance active* | *46,207 %* |
| *PDRE PILOSELLE BIO* | *Substance active* | *39,310 %* |
| *FIBRE POMME BIO* | *Excipient (agent de charge et anti-agglomérant*/*stabilisant)* | *14,483 %* |

### 6.2 Observations à partir des profils radar de chacune des deux substances actives

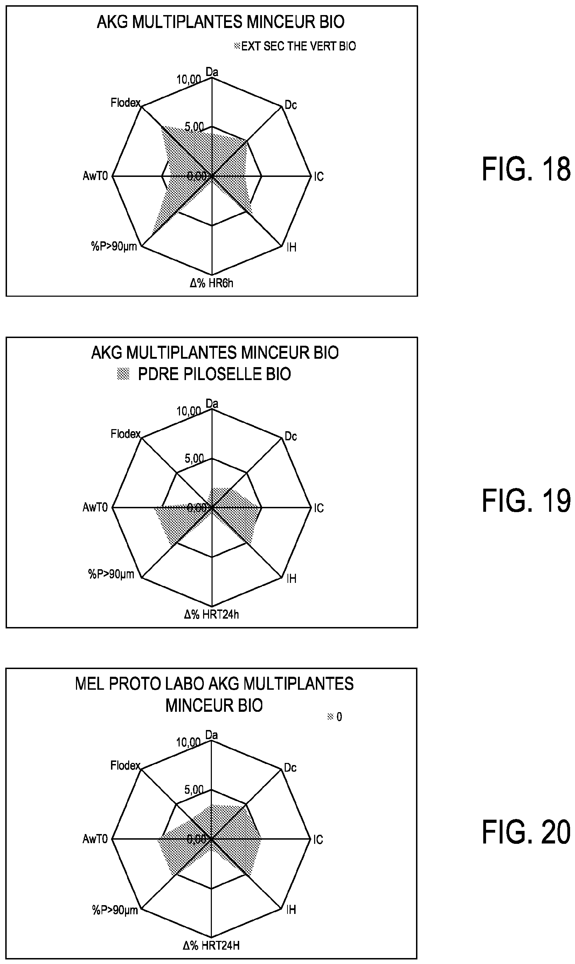

Les profils radar du thé vert bio et de la poudre de piloselle bio sont respectivement présentés en figures 18 et 19.

L'extrait sec de thé vert bio présente intrinsèquement une aptitude à l'écoulement intéressante mais est sensible à la chaleur et à l'humidité. L'extrait change d'état et fond au bout de 24 heures en enceinte climatique à 40°C et 75%HR, rendant impossible toute mesure d'Aw ou de teneur en eau. Le profil radar est donc construit avec les mesures réalisées à T6h00 (cf. figure 18). L'extrait sec de thé vert est donc fortement hygroscopique, et, par conséquent, doit être protégé par un agent stabilisant/anti-agglomérant afin d'en préserver les propriétés physiques et chimiques.

La poudre de piloselle, quant à elle, ne semble pas sensible à l'humidité, donc pas hygroscopique, mais n'apparaît pas posséder de propriétés pharmaco-techniques suffisamment satisfaisantes notamment en termes d'écoulement.

Le profil radar réalisé à partir du produit objet du présent exemple est présenté en figure 20. Ce profil radar s'avère globalement satisfaisant, l'écoulement étant correct et le mélange restant stable pendant 24h en boîte de Pétri à 40°C/75%HR.

### Références bibliographiques

[1] J. M. Sune Negre, et al. «Application of the SeDeM Diagram and a new mathematical équation in the design of direct compression tablet formulation ». European Journal of Pharmaceutics and Biopharmaceutics 69 (2008) 1029-1039.
[2] J. M. Sune Negre, et al. « SeDeM Diagram : A New Expert System for the Formulation of Drugs in Solid Form ». Chapter 2 of « Expert Systems for Human, Materials and Automation », 2011
[3] A GIOIA « Intrinsic flowability : a new tecHnology for powder-flowability classification ». HANSON RESEARCH from Pharmaceutical Technology Feb. 1980

## Revendications

1. Composition à usage pharmaceutique, vétérinaire, pour dispositif médical ou pour complément alimentaire, ladite composition se présentant sous forme solide, de préférence sous forme pulvérulente, et comprenant :
- au moins une substance active, de préférence d'origine naturelle, préférablement d'origine naturelle végétale ou d'origine naturelle issue de la ruche, et
- au moins un excipient à usage pharmaceutique, vétérinaire, pour dispositif médical ou pour complément alimentaire ;
ledit excipient se présentant sous forme solide et comprenant un ensemble de fibres alimentaires végétales non modifiées chimiquement, dans un pourcentage massique supérieur ou égal à 50% par rapport à la masse totale de l'excipient, ledit ensemble de fibres alimentaires comprenant :
- un pourcentage massique de fibres solubles dans l'eau inférieur à 20%, de préférence inférieur ou égal à 15%, par exemple inférieur ou égal à 10%, par rapport à la masse totale de l'excipient, et
- un pourcentage massique de fibres insolubles dans l'eau supérieur à 40%, de préférence supérieur ou égal à 45%, par rapport à la masse totale de l'excipient ;
ledit excipent présentant avantageusement un pourcentage massique en eau inférieur ou égal à 8% par rapport à la masse totale de l'excipient ;
ledit excipient se présentant sous forme pulvérulente, ledit excipient sous forme pulvérulente étant sous forme de particules de taille micronique telles que des particules micronisées, lesdites particules de taille micronique ayant une granulométrie inférieure ou égale à 1000 µm, préférablement inférieure à 1000 µm, et se situant préférentiellement entre environ 1 µm et environ 250 µm.

2. Composition selon la revendication précédente, dans laquelle ledit ensemble de fibres alimentaires comprend des fibres de plante(s), de fruit(s) et/ou des fibres de céréale(s), de préférence des fibres de pomme, d'avoine, d'aronia, de cassis, de pois et/ou de sarrasin.

3. Composition selon la revendication 1 ou 2, dans laquelle ledit excipient sous forme pulvérulente a la distribution granulométrique suivante :
- Dv10 < 20 µm, de préférence < 15 µm,
- Dv50 < à 100 µm, de préférence < à 50 µm, et/ou
- Dv90 < à 250 µm, de préférence < à 200 µm ;
de manière préférée ladite distribution granulométrique étant la suivante :
- Dv90 < à 250 µm, de préférence < à 200 µm, et
- Dv50 < à 100 µm, de préférence < à 50 µm, et/ou Dv10 < 20 µm, de préférence < 15 µm ;
de manière particulièrement préférée ladite distribution granulométrique étant la suivante :
- Dv90 < à 250 µm, de préférence < à 200 µm, et
- Dv50 < à 100 µm, de préférence < à 50 µm, et Dv10 < 20 µm, de préférence < 15 µm.

4. Composition selon l'une des revendications 1 à 3, dans laquelle ledit ensemble de fibres alimentaires végétales non modifiées chimiquement est présent dans un pourcentage massique supérieur ou égal à 60% par rapport à la masse totale de l'excipient.

5. Composition selon l'une des revendications 1 à 4, dans laquelle ladite au moins une substance active est une substance hygroscopique.

6. Composition selon l'une des revendications 1 à 5, dans laquelle ladite au moins une substance active est une préparation à base de plante(s) sous forme de poudre et/ou une substance active d'origine naturelle issue de la ruche telle que le miel, la gelée royale ou la propolis sous forme de poudre ; de manière préférée ladite poudre ayant une granulométrie inférieure à 1000 µm et se situant préférentiellement entre 1 et 250 µm.

7. Forme galénique solide comprenant la composition selon l'une des revendications 1 à 6, ladite forme galénique solide étant sélectionnée parmi :
- un comprimé, tel qu'un comprimé à avaler, à croquer, effervescent, orodispersible,
- une pastille,
- une poudre, telle qu'une poudre en sachet, en stick, vrac, une gélule ;
ladite forme galénique solide étant préférablement sélectionnée parmi un comprimé ou une gélule ; de manière particulièrement préférée ladite forme galénique solide étant une gélule.

8. Médicament à usage humain ou animal, dispositif médical ou complément alimentaire comprenant la composition selon l'une des revendications 1 à 6 ou la forme galénique solide selon la revendication 7.

9. Utilisation d'un excipient tel que défini dans l'une des revendications 1 à 4 en tant qu'agent de charge, agent stabilisant et/ou agent anti-agglomérant, de préférence en tant qu'agent anti-agglomérant, avantageusement en tant qu'agent anti-agglomérant sélectionné parmi les agents protecteurs contre l'humidité, dans une composition pharmaceutique, vétérinaire, un dispositif médical ou un complément alimentaire.

10. Utilisation d'un ensemble de fibres alimentaires végétales non modifiées chimiquement pour la préparation d'un excipient tel que défini dans l'une des revendications 1 à 3, ledit ensemble de fibres alimentaires comprenant :
- un pourcentage massique de fibres solubles dans l'eau inférieur à 20%, de préférence inférieur ou égal à 15%, par exemple inférieur à 10%, par rapport à la masse totale de l'excipient, et
- un pourcentage massique de fibres insolubles dans l'eau supérieur à 40%, de préférence supérieur ou égal à 45%, par rapport à la masse totale de l'excipient ;
ledit ensemble de fibres alimentaires étant utilisé dans un pourcentage massique supérieur ou égal à 50% par rapport à la masse totale de l'excipient.

11. Utilisation selon la revendication 10, ledit ensemble de fibres alimentaires étant utilisé dans un pourcentage massique supérieur ou égal à 60% par rapport à la masse totale de l'excipient.

## Patentansprüche

1. Eine Zusammensetzung zur pharmazeutischen, veterinärmedizinischen Verwendung, für ein medizinisches Gerät oder zur Nahrungsergänzung, wobei die Zusammensetzung in fester Form, vorzugsweise in Pulverform, vorliegt und Folgendes beinhaltet:
- mindestens einen Wirkstoff, vorzugsweise natürlichen Ursprungs, bevorzugt natürlichen pflanzlichen Ursprungs oder natürlichen Ursprungs aus dem Bienenstock, und
- mindestens einen Hilfsstoff zur pharmazeutischen, veterinärmedizinischen Verwendung, für ein medizinisches Gerät oder für ein Nahrungsergänzungsmittel;
wobei der Hilfsstoff in fester Form vorliegt und eine Reihe von chemisch nicht modifizierten pflanzlichen Ballaststoffen in einem Massenanteil von mehr als oder gleich 50 %, bezogen auf die Gesamtmasse des Hilfsstoffs, beinhaltet, wobei die Reihe von Ballaststoffen Folgendes beinhaltet:
- einen Massenanteil von wasserlöslichen Fasern von weniger als 20 %, vorzugsweise weniger als oder gleich 15 %, zum Beispiel weniger als oder gleich 10 %, bezogen auf die Gesamtmasse des Hilfsstoffs, und
- einen Massenanteil von wasserunlöslichen Fasern von mehr als 40 %, vorzugsweise mehr als oder gleich 45 %, bezogen auf die Gesamtmasse des Hilfsstoffs;
wobei der Hilfsstoff vorteilhafterweise einen Massenanteil von Wasser von weniger als oder gleich 8 %, bezogen auf die Gesamtmasse des Hilfsstoffs, aufweist;
wobei der Hilfsstoff in Pulverform vorliegt, wobei der Hilfsstoff in Pulverform in Form von Partikeln mit Mikrometergröße, wie mikronisierten Partikeln, vorliegt, wobei die Partikel mit Mikrometergröße eine Korngröße von weniger als oder gleich 1000 µm, bevorzugt weniger als 1000 µm, aufweisen und vorzugsweise zwischen etwa 1 µm und etwa 250 µm liegen.

2. Zusammensetzung gemäß dem vorhergehenden Anspruch, wobei die Reihe von Ballaststoffen Pflanzen-, Fruchtfasern und/oder Getreidefasern beinhaltet, vorzugsweise Apfel-, Hafer-, Aronia-, Cassis-, Erbsen- und/oder Buchweizenfasern.

3. Zusammensetzung gemäß Anspruch 1 oder 2, wobei der Hilfsstoff in Pulverform die folgende Korngrößenverteilung aufweist:
- Dv10 < 20 µm, vorzugsweise < 15 µm,
- Dv50 < 100 µm, vorzugsweise < 50 µm, und/oder
- Dv90 < 250 µm, vorzugsweise < 200 µm;
wobei auf bevorzugte Weise die Korngrößenverteilung wie folgt ist:
- Dv90 < 250 µm, vorzugsweise < 200 µm, und
- Dv50 < 100 µm, vorzugsweise < 50 µm, und/oder Dv10 < 20 µm, vorzugsweise < 15 µm;
wobei auf besonders bevorzugte Weise die Korngrößenverteilung wie folgt ist:
- Dv90 < 250 µm, vorzugsweise < 200 µm, und
- Dv50 < 100 µm, vorzugsweise < 50 µm, und Dv10 < 20 µm, vorzugsweise < 15 µm.

4. Zusammensetzung gemäß einem der Ansprüche 1 bis 3, wobei die Reihe von chemisch nicht modifizierten pflanzlichen Ballaststoffen in einem Massenanteil von mehr als oder gleich 60 % vorhanden ist, bezogen auf die Gesamtmasse des Hilfsstoffs.

5. Zusammensetzung gemäß einem der Ansprüche 1 bis 4, wobei der mindestens eine Wirkstoff ein hygroskopischer Stoff ist.

6. Zusammensetzung gemäß einem der Ansprüche 1 bis 5, wobei der mindestens eine Wirkstoff ein Präparat auf Pflanzenbasis in Pulverform und/oder ein Wirkstoff natürlichen Ursprungs aus dem Bienenstock wie Honig, Gelée Royale oder Propolis in Pulverform ist; wobei das Pulver auf bevorzugte Weise eine Korngröße von weniger als 1000 µm aufweist und vorzugsweisezwischen 1 und 250 µm liegt.

7. Eine feste Darreichungsform, die die Zusammensetzung gemäß einem der Ansprüche 1 bis 6 beinhaltet, wobei die feste Darreichungsform aus Folgendem ausgewählt ist:
- einer Tablette, wie etwa einer Schluck-, Kau-, Brause-, Schmelztablette,
- einer Pastille,
- einem Pulver, wie etwa einem Pulver in einem Sachet, in einem Stick, lose, in einer Kapsel;
wobei die feste Darreichungsform bevorzugt aus einer Tablette oder einer Kapsel ausgewählt wird; wobei auf besonders bevorzugte Weise die feste Darreichungsform eine Kapsel ist.

8. Ein Arzneimittel zur Verwendung bei Menschen oder Tieren, medizinisches Gerät oder ein Nahrungsergänzungsmittel, das die Zusammensetzung gemäß einem der Ansprüche 1 bis 6 oder die feste Darreichungsform gemäß Anspruch 7 beinhaltet.

9. Eine Verwendung eines Hilfsstoffs wie in einem der Ansprüche 1 bis 4 definiert, als Füllstoff, Stabilisierungsmittel und/oder Antiklumpmittel, vorzugsweise als Antiklumpmittel, vorteilhafterweise als Antiklumpmittel, das ausgewählt ist aus Feuchtigkeitsschutzmitteln, in einer pharmazeutischen, veterinärmedizinischen Zusammensetzung, einem medizinischen Gerät oder einem Nahrungsergänzungsmittel.

10. Eine Verwendung einer Reihe von chemisch nicht modifizierten pflanzlichen Ballaststoffen für die Herstellung eines Hilfsstoffs, wie in einem der Ansprüche 1 bis 3 definiert, wobei die Reihe von Ballaststoffen Folgendes beinhaltet:
- einen Massenanteil von wasserlöslichen Fasern von weniger als 20 %, vorzugsweise weniger als oder gleich 15 %, zum Beispiel weniger als 10 %, bezogen auf die Gesamtmasse des Hilfsstoffs, und
- einen Massenanteil an wasserunlöslichen Fasern von mehr als 40 %, vorzugsweise mehr als oder gleich 45 %, bezogen auf die Gesamtmasse des Hilfsstoffs;
wobei die Reihe von Ballaststoffen in einem Massenanteil von mehr als oder gleich 50 %, bezogen auf die Gesamtmasse des Hilfsstoffs, verwendet wird.

11. Verwendung gemäß Anspruch 10, wobei die Reihe von Ballaststoffen in einem Massenanteil von mehr als oder gleich 60 %, bezogen auf die Gesamtmasse des Hilfsstoffs, verwendet wird.

## Claims

1. A composition for pharmaceutical, veterinary use, for a medical device, or for a dietary supplement, said composition presenting a solid form, preferably in powdery form, and comprising:
- at least one active substance, preferably of natural origin, preferably of plant natural origin or of natural origin from a beehive, and
- at least one excipient for pharmaceutical, veterinary use for a medical device, or for a dietary supplement;
said excipient presenting a solid form and comprising a set of chemically unmodified dietary plant fibres, in a mass percentage greater than or equal to 50% relative to the total mass of the excipient, said set of dietary fibres comprising:
- a mass percentage of water-soluble fibres less than 20%, preferably less than or equal to 15%, for example less than or equal to 10%, relative to the total mass of the excipient, and
- a mass percentage of water-insoluble fibres greater than 40%, preferably greater than or equal to 45%, relative to the total mass of the excipient;
said excipient advantageously presenting a mass percentage in water less than or equal to 8% relative to the total mass of the excipient;
said excipient presenting a powdery form, said powdery-form excipient being in the form of micron-sized particles such as micronised particles, said micron-sized particles having a granulometry less than or equal to 1000 µm, preferably less than 1000 µm, and preferentially being between about 1 µm and about 250 µm.

2. The composition according to the preceding claim, wherein said set of dietary fibres comprises fibres from plant(s), fruit(s) and/or fibres from grain(s), preferably apple, oat, chokeberry, blackcurrant, pea and/or buckwheat fibres.

3. The composition according to claim 1 or 2, wherein said powdery-form excipient has the following granulometric distribution:
- Dv10 < 20 µm, preferably < 15 µm,
- Dv50 < 100 µm, preferably < 50 µm, and/or
- Dv90 < 250 µm, preferably < 200 µm,
in a preferred manner said granulometric distribution being as follows:
- Dv90 < 250 µm, preferably < 200 µm, and
- Dv50 < 100 µm, preferably < 50 µm, and/or Dv10 < 20 µm, preferably < 15 µm;
in a particularly preferred manner said granulometric distribution being as follows:
- Dv90 < 250 µm, preferably < 200 µm, and
- Dv50 < 100 µm, preferably < 50 µm, and Dv10 < 20 µm, preferably < 15 µm.

4. The composition according to one of claims 1 to 3, wherein said set of chemically unmodified dietary plant fibres is present in a mass percentage greater than or equal to 60% relative to the total mass of the excipient.

5. The composition according to one of claims 1 to 4, wherein said at least one active substance is a hygroscopic substance.

6. The composition according to one of claims 1 to 5, wherein said at least one active substance is a plant-based preparation in powder form and/or an active substance of natural origin from a beehive, such as honey, royal jelly or propolis in powder form; in a preferred manner said powder having a granulometry less than 1000 µm and preferentially being between 1 and 250 µm.

7. A solid dosage form comprising the composition according to one of claims 1 to 6, said solid dosage form being selected from:
- a tablet, such as a swallowable, chewable, effervescent, orodispersible tablet,
- a lozenge,
- a powder, such as a powder in a sachet, a stick, loose, in a capsule;
said solid dosage form being preferably selected from a tablet or a capsule; in a particularly preferred manner said solid dosage form being a capsule.

8. A medicine for human or animal use, a medical device, or dietary supplement comprising the composition according to one of claims 1 to 6 or the solid dosage form according to claim 7.

9. Use of an excipient such as defined in one of claims 1 to 4 as a bulking agent, stabilising agent and/or an anti-caking agent, preferably as an anti-caking agent, advantageously as an anti-caking agent selected from agents which offer protection from humidity, in a pharmaceutical, veterinary composition, a medical device, or a dietary supplement.

10. Use of a set of chemically unmodified dietary plant fibres for the preparation of an excipient such as defined in one of claims 1 to 3, said set of dietary fibres comprising:
- a mass percentage of water-soluble fibres less than 20%, preferably less than or equal to 15%, for example less than 10%, relative to the total mass of the excipient, and
- a mass percentage of water-soluble fibres greater than 40%, preferably greater than or equal to 45%, relative to the total mass of the excipient;
said set of dietary fibres being used in a mass percentage greater than or equal to 50% relative to the total mass of the excipient.

11. Use according to claim 10, said set of dietary fibres being used in a mass percentage greater than or equal to 60% relative to the total mass of the excipient.
